(19) Europäisches Patentamt
European Patent Office
Office européen des brevets

(11) Veröffentlichungsnummer: **0 135 476**
**B1**

# (12) EUROPÄISCHE PATENTSCHRIFT

(45) Veröffentlichungstag der Patentschrift: **14.09.88**

(51) Int. Cl.⁴: **C 07 J 3/00**, A 61 K 31/56 // C07J71/00

(21) Anmeldenummer: **84810397.4**

(22) Anmeldetag: **13.08.84**

(54) **Steroid Carbonsäureester.**

(30) Priorität: **18.08.83 CH 4512/83**

(43) Veröffentlichungstag der Anmeldung: **27.03.85 Patentblatt 85/13**

(45) Bekanntmachung des Hinweises auf die Patenterteilung: **14.09.88 Patentblatt 88/37**

(84) Benannte Vertragsstaaten: **AT BE CH DE FR GB IT LI LU NL SE**

(56) Entgegenhaltungen:
**CH - A - 634 081**
**FR - A - 2 122 539**
**FR - A - 2 282 899**
**US - A - 4 285 937**

(73) Patentinhaber: **CIBA-GEIGY AG, Klybeckstrasse 141, CH-4002 Basel (CH)**

(72) Erfinder: **Schmidlin, Julius, Dr., Leimenstrasse 39, CH-4051 Basel (CH)**

**EP 0 135 476 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft neue steroidale Carbonsäureester, insbesondere Methylester von 17β-Carbonsäuren der Androstan-Reihe, entsprechend der Formel

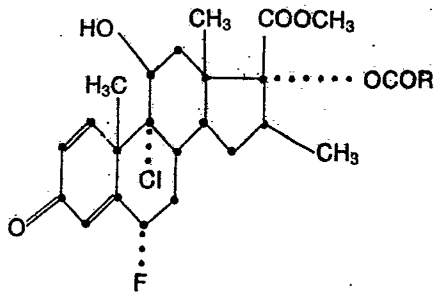
(A),

worin R ein Alkyl mit 1 bis 4 Kohlenstoffatomen bedeutet, und Verfahren zu ihrer Herstellung, sowie auch pharmazeutische Zusammensetzungen enthaltend diese Verbindungen und Verfahren zur Herstellung derselben.

Das durch das Symbol R charakterisierte Alkyl ist vorzugsweise linear, wie Methyl, Propyl, Butyl und vor allem Ethyl.

Besonders hervorzuheben unter diesen Verbindungen sind Methylester der
9α-Chlor-6α-fluor-11β-hydroxy-16β-methyl-3-oxo-17α-acetoxy-androsta-1,4-dien-17β-carbonsäure,
der 9α-Chlor-6α-fluor-11β-hydroxy-16β-methyl-3-oxo-17α-valeryloxy-androsta-1,4-dien-17β-carbonsäure und vor allem der
9α-Chlor-6α-fluor-11β-hydroxy-16β-methyl-3-oxo-17α-propionyloxy-androsta-1,4-dien-17β-carbonsäure-methylester der Formel

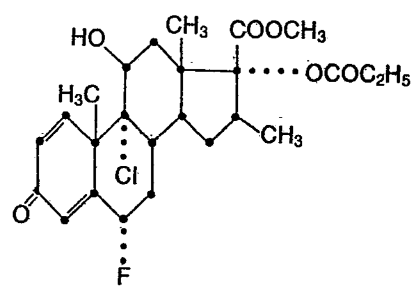
(I).

Ester mehrfach halogenierter Androsta-1,4-dien-3-on-17β-carbonsäuren sind beschrieben worden. So betrifft z.B. die U.S. Patentschrift Nr. 4 285 937 Ester ein-, zwei- und dreiwertiger aliphatischer Alkohole mit
11β,17α-Dihydroxy-3-oxo-androsta-1,4-dien-17β-carbonsäuren,
deren 17α-Hydroxyl durch eine aliphatische Carbonsäure mit höchstens 7 Kohlenstoffatomen acyliert ist und worin sich in 2-Stellung Chlor oder Wasserstoff, in 6α- und/oder 9α-Stellung unabhängig voneinander Chlor oder Fluor und in 16-Stellung ein α- oder β-orientiertes Methyl oder aber Methylen befindet. Unter diesen breiten allgemeinen Umfang kann man auch die Verbindungen der vorliegenden Erfindung subsummieren, obwohl sie sonst in keiner Weise näher berücksichtigt worden sind und das Hauptgewicht der Patentschrift bei einer anders substituierter Verbindungsgruppe liegt. Besonders hervorgehoben sind dort (vgl. Ansprüche 15 und 16) Niederalkylester von Säuren, die als spezifisches Merkmal das 16α-Methyl aufweisen und als weitere Merkmale eine durch Niederalkanoyl acylierte 17α-Hydroxygruppe, 6α-Fluor und 9α-Chlor tragen und worin die 2-Stellung unsubstituiert ist. Ihrer besonderen Wirksamkeit wegen werden die folgenden Verbindungen spezifisch genannt (siehe Spalte 2, Zeilen 32–41 der obengenannten Patentschrift):
2-Chlor-6α,9α-difluor-11β-hydroxy-17α-propionyloxy-16α-methyl-3-oxo-androsta-1,4-dien-17β-carbonsäure-methylester,
2,9α-Dichlor-6α-fluor-11β-hydroxy-17α-propionyloxy-16α-methyl-3-oxo-androsta-1,4-dien-17β-carbonsäure-methylester,
sowie auch der in 2-Stellung chlorfreie
9α-Chlor-6α-fluor-11β-hydroxy-17α-propionyloxy-16α-methyl-3-oxo-androsta-1,4-dien-17β-carbonsäure-methylester (O).

Bei den übrigen 2 in der Patentschrift erwähnten Verbindungen, und zwar
2-Chlor-9α-fluor-11β-hydroxy-17α-propionyloxy-16β-methyl-3-oxo-androsta-1,4-dien-17β-carbonsäure-methylester und
2,9α-Dichlor-6α-fluor-11β-hydroxy-17α-propionyloxy-16α-methyl-3-oxo-androsta-1,4-dien-17β-carbonsäure-chlormethylester,
wurden keine besonderen Vorteile angegeben. – In der erwähnten Patentschrift wird berichtet, dass die dort offenbarten Ester eine hohe antiinflammatorische Wirksamkeit besitzen (z.B. weisen sie im Fremdkörpergranulom-Test an der Ratte eine ausgeprägte antiinflammatorische Wirkung im Dosisbereich von ca. 0,001–0,03 mg pro Rohwattepressling auf); bei derselben Verabreichung tritt aber auch ihre Wirkung auf Thymus, Nebennieren und das Körpergewicht auf, allerdings erst ab Dosen von 0,3 mg pro Rohwattepressling. Auch im Rattenohr-Test nach Tonelli erwiesen sich die Verbindungen der genannten Patentschrift als besonders wirksam.

Trotz der wesentlich geringeren systemischen Wirkung, die bei den getesteten Verbindungen der genannten Patentschrift im Vergleich zu vorbekannten Wirkstoffen erreicht worden ist, verblieb die Suche nach topisch hochwirksamen Corticoiden, welche dabei aber eine möglichst geringe oder sogar ausbleibende systemische Wirksamkeit, und zwar auch bei längerer Verabreichung besässen, eine der vordringlichsten Aufgaben auf diesem Gebiet. Obwohl sich die hervorgehobenen 16α-Methyl-Verbindungen in der angewendeten Testanordnung als zufriedenstellend erweisen, vermögen sie härteren Ansprüchen, insbesondere bei direkter Prüfung ihrer systemischer Effekte, nicht zu genügen.

Überraschenderweise hat sich nun aber gezeigt, dass sich aus der allgemein umfassten, breiten Verbindungsklasse der diskutierten Patentschrift eine bisher nicht beachtete enge Auswahl von Verbindungen heraushebt, die vor allem

durch die β-Orientierung des 16-Methyls charakterisiert ist und sich durch eine erstaunlich niedrige systemische Wirksamkeit auszeichnet, wie es anhand der folgenden Datenzusammenstellung gezeigt werden kann:

Tabelle 1: Rohwattegranulom-Test (Ratte) lokal

| Verbindung | Hemmung bzw. Reduktion ($ED_{20-30}$ in µg pro Pressling) Granulom | kontralaterales Granulom | Nebennieren | Thymus | Körpergewicht |
|---|---|---|---|---|---|
| I | 0,2 | 125 ∅* | 125 ∅ | 125 ∅ | 125 ∅ |
| O | 0,2 | 125 ∅ | 125 ∅ | 125 ∅ | 125 ∅ |

* ∅ = ohne Effekt in der angegebenen Dosis

Tabelle 2: Rohwattegranulom-Test (Ratte) peroral

| Verbindung | Hemmung bzw. Reduktion ($ED_{20-30}$ in mg pro kg Tier Granulom | Nebennieren | Thymus | Körpergewicht |
|---|---|---|---|---|
| I | 3 ∅* | 3 ∅ | 3 ∅ | 3 ∅ |
| O | 3 | 3 | 1 | 3 |

* ∅ = ohne Effekt in der angegebenen Dosis

Tabelle 3: Leberglykogen-Test (Ratte) subcutan
Tryptophanpyrrolase-Test (Ratte) subcutan

| Verbindung | Glykogenbildung ($ED_{20-30}$ in mg pro kg Tier) | Induktion der Tryptophanpyrrolase ($ED_{20}$ in mg pro kg Tier) |
|---|---|---|
| I | >10 | >3 |
| O | 1 | 0,1–0,3 |

Gemäss Tabelle 1 weist die 16β-Methyl-Verbindung (I) topisch eine starke antiinflammatorische Wirkung auf, die derjenigen der entsprechenden 16α-Methyl-Verbindung (O) gleichkommt. Die genannte 16β-Methyl-Verbindung (I) unterscheidet sich jedoch, wie aus Tabelle 2 hervorgeht, vorteilhaft von der 16α-Methyl-Verbindung (O), indem sie bei peroraler Applikation den Thymus, die Nebennieren und das Körpergewicht in der geprüften Dosierung nicht beeinflusst. Für das weitgehende Fehlen corticosteroid-spezifischer Nebenwirkungen spricht vor allem der aus der Tabelle 3 ersichtliche Befund, wonach die erfindungsgemässe 16β-Methyl-Verbindung (I) nach subcutaner Verabreichung an der Ratte, im Unterschied zur 16α-Methyl-Verbindung (O), selbst in hohen Dosen keine signifikante Vermehrung des Leberglykogens bewirkt. In Übereinstimmung damit ist der ebenfalls in Tabelle 3 ausgewiesene markante Unterschied, welcher für das vorerwähnte Verbindungspaar (I) und (O) bezüglich der Induktion der Tryptophanpyrrolase gefunden wird. In diesem Test zeigt die erfindungsgemässe 16β-Methyl-Verbindung (I) im Vergleich zur 16α-Methyl-Verbindung (O) selbst bei einer 10 bis 30 mal höheren Dosierung den für systemisch wirksame Glucocorticoide spezifischen Effekt nicht.

Dank diesem überraschenden, experimentell nachgewiesenen Ausbleiben jeglicher systemischer Nebenwirkungen bei vollem Beibehalten der ausserordentlich hohen lokalen antiinflammatorischen Wirksamkeit sind die erfindungsgemässen Verbindungen der Formel (A) einwandfrei geeignet nicht nur als lokal, vor allem topisch, anwendbare entzündungshemmende Heilmittel für alle Indikationen, welche bei Corticoiden üblich sind, sondern überdies hinaus auch für spezielle Anwendungsgebiete, in welchen bekannte Corticoide oft schwerwiegende Nachteile aufweisen, beispielsweise für langdauernde Therapie hartnäckiger und/oder rezidivierender Hauterkrankungen, wie Psoriasis. Ausserdem können die erfindungsgemässen Verbindungen für Verabreichung durch Inhalation zur therapeutischen Behandlung asthmatischer Zustände und Symptome verwendet werden.

Die neuen erfindungsgemässen Ester der oben charakterisierten Formel (A) können in an sich bekannter Weise hergestellt werden, insbesondere dadurch, dass man

a) eine 9β,11β-Oxoverbindung der Formel

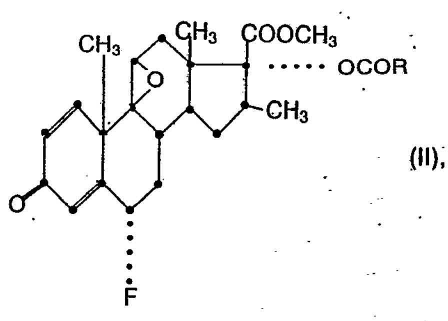

(II),

worin R die oben genannte Bedeutung hat, mit Chlorwasserstoff oder einem Chlorwasserstoff-abgebenden Mittel behandelt, oder

b) an eine 9(11)-ungesättigte Verbindung der Formel

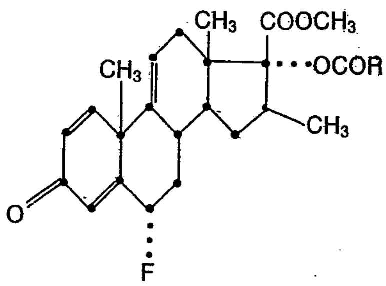

(III)

worin R die oben genannte Bedeutung hat, Elemente der Hypochlorigen Säure anlagert, oder

c) eine 1,2-gesättigte Verbindung der Formel

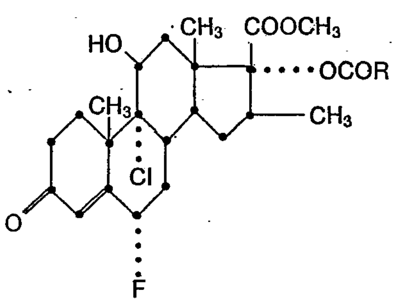

(IV)

worin R die oben genannte Bedeutung hat, in 1,2-Stellung dehydriert, oder

d) eine Carbonsäure der Formel

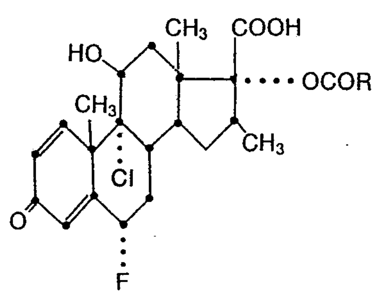

(V)

worin R die oben genannte Bedeutung hat, oder ein Salz oder ein reaktionsfähiges funktionelles Derivat davon, mit Hilfe eines methylierenden Mittels verestert, oder

e) den 9α-Chlor-6α-fluor-11β,17α-dihydroxy-16β-methyl-3-oxo-androsta-1,4-dien-17β-carbonsäure-methylester der Formel

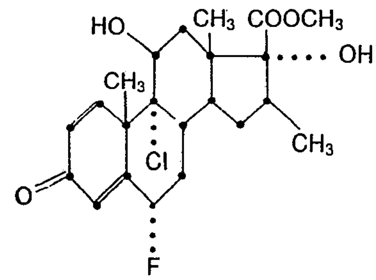

(VI)

mit einer Carbonsäure der Formel R-COOH (VII), worin R die oben genannte Bedeutung hat, oder einem reaktiven funktionellen Derivat davon, gegebenenfalls unter vorübergehendem Schutz des 11β-Hydroxyls, acyliert, oder

f) in einer dem oben definierten Methylester der Formel (A) entsprechenden Verbindung mit geschütztem 11β-Hydroxyl der Formel

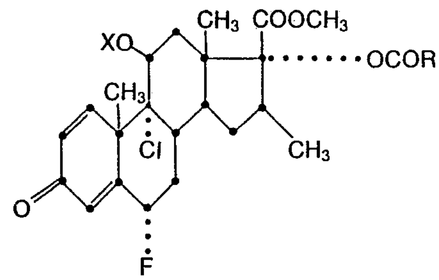

(VIII),

worin X eine Hydroxyl-Schutzgruppe darstellt und R die oben genannte Bedeutung hat, die Schutzgruppe X abspaltet.

Die erfindungsgemässe Aufspaltung der 9β,11β-Oxidogruppe in einem Ausgangsmaterial der Formel (II) gemäss Verfahrensvariante a) durch Behandeln mit Chlorwasserstoff wird in an sich bekannter Weise vorgenommen, wobei man zweckmässig wasserfreien Chlorwasserstoff, gegebenenfalls in Gegenwart eines inerten Lösungsmittels, wie Chloroform, Tetrahydrofuran oder insbesondere Dimethylformamid, verwendet.

Anstelle des Halogenwasserstoffs selber kann man auch ein Chlorwasserstoff-abgebendes Mittel, wie dessen Salz mit einer tertiären organischen Base, z.B. Pyridin, verwenden.

Die Ausgangsstoffe der Formel II können in an sich bekannter Weise erhalten werden, z.B. durch Abspalten von Wasser aus einem

6α-Fluor-11β-hydroxy-16β-methyl-17α-OCOR-3-oxo-androsta-1,4-dien-17β-carbonsäure-methylester,

z.B. durch Behandeln mit einem geeigneten Dehydratisierungsmittel, z.B. einem Säurechlorid, wie Phosphoroxychlorid oder Methansulfonsäurechlorid, in Gegenwart einer Base, z.B. Pyridin, Anlagern von unterbromiger Säure (die z.B. in Form von N-Bromacetamid oder N-Bromsuccinimid in saurem Milieu eingesetzt wird) an die 9,11-Doppelbindung des so gebildeten

6α-Fluor-16β-methyl-17α-OCOR-3-oxo-androsta-1,4,9(11)-trien-17β-carbonsäure-methylesters

und Abspalten von Bromwasserstoff aus dem entstandenen 9α-Brom-11β-hydrin durch Behandeln

mit einer Base, insbesondere einem Alkalimetallsalz einer Carbonsäure, z.B. Kaliumacetat, oder einer aprotischen organischen Base, vornehmlich z.B. mit 1,5-Diazabicyclo[5,4,0]undec-5-en, unter Bildung des gewünschten Ausgangsmaterials der Formel II. Man kann zur Abspaltung von Bromwasserstoff anorganische Basen, wie Alkalimetallhydroxide oder -carbonate, z.B. Natriumhydroxid oder Kaliumcarbonat, oder aber Alkalimetallalkanolate, wie Natriummethylat oder Kalium-tert-butylat, einsetzen; in diesem Fall werden jedoch, mindestens teilweise, die 17α-Alkanoyloxygruppe und die 17β-Methoxycarbonylgruppe zur freien Hydroxyl- bzw. Carboxylgruppe verseift und müssen nachträglich, z.B. unter Bedingungen der weiter unten beschriebenen Verfahrensvarianten d) und e) verestert werden. In den obigen Zwischenprodukten hat R die vorstehend gegebene Bedeutung. Analog können auch die entsprechenden 1,2-gesättigten Ausgangsstoffe erhalten werden.

Die erfindungsgemässe Anlagerung der Elemente der Hypochlorigen Säure an die 9(11)-Doppelbindung des Ausgangsstoffes der Formel III gemäss Verfahrensvariante b) erfolgt in an sich bekannter Weise. Dabei arbeitet man z.B. mit wässriger Hypochloriger Säure, oder man kann ein die Hypochlorige Säure abgebendes Mittel, wie ein N-Chlor-carbonsäureamid oder -imid, z.B. N-Chlorsuccinimid, (vgl. US-Patentschrift 3 057 886), verwenden. Die Reaktion führt man in einem inerten Lösungsmittel, wie einem tertiären Alkohol, z.B. tert-Butylalkohol, einem Ether, z.B. Diethylether, 1,2-Dimethoxyethan, Dioxan oder Tetrahydrofuran, oder einem Keton, z.B. Aceton, in Gegenwart von Wasser und gegebenenfalls einer Säure durch. – Die Anlagerung kann aber auch in nicht-wässrigem Medium erfolgen. Eine besonders vorteilhafte Ausführungsform stellt dabei die Verwendung von Niederalkylhypochloriten, in erster Linie von tert-Butylhypochlorit, in tert-Butylalkohol oder einem inerten, mit Wasser nicht mischbaren Lösungsmittel, wie einem Nitrokohlenwasserstoff, z.B. Nitromethan, üblicherweise in Gegenwart von Perchlorsäure dar (vgl. Deutsche Patentschrift 2 011 559).

Die Ausgangsstoffe der Formel III bzw. ihre 1,2-Dihydroanalogen, können in an sich bekannter Weise hergestellt werden, z.B. durch Abspalten von Wasser aus einem
6α-Fluor-11β-hydroxy-16β-methyl-17α-OCOR-3-oxo-(androsta-1,4-dien
bzw. androst-4-en)-17β-carbonsäure-methylester
z.B. durch Behandeln mit einem geeigneten Dehydratisierungsmittel, wie einem Säurechlorid, z.B. Phosphoroxychlorid oder Methansulfonsäurechlorid, in Gegenwart einer Base, z.B. Pyridin. (Im obigen Ausgangsstoff hat R die eingangs angegebene Bedeutung.)

Die erfindungsgemässe 1,2-Dehydrierung gemäss Verfahrensvariante c) kann in an sich bekannter Weise z.B. durch biologische Dehydrierungsverfahren erfolgen, z.B. mittels der Mikroorganismen Corynebacterium simplex oder Septomyxa affinis oder ihrer Enzymsysteme, oder mit Selendioxid in einem organischen Lösungsmittel, z.B. tert-Butylalkohol. Vorzugsweise lässt man jedoch 2,3-Dichlor-5,6-dicyan-1,4-benzochinon, zweckmässig bei Temperaturen von etwa 40 °C bis Siedehitze während mehreren, z.B. 6–24 Stunden einwirken; als Reaktionsmedium verwendet man übliche organische Lösungsmittel, z.B. aromatische Kohlenwasserstoffe, wie Benzol oder Xylol, niederaliphatische Alkohole, wie Ethanol, Propylalkohol oder tert-Butylalkohol, niederaliphatische Ketone, wie Aceton oder 2-Butanon, aliphatische Ester, wie Ethylacetat, oder cyclische Ether, wie Dioxan oder Tetrahydrofuran. Alle diese Varianten sind Standardverfahren der Steroidchemie und sind mithin allgemein bekannt.

Die Ausgangsstoffe der Formel IV können z.B. erhalten werden, indem man an einen
6α-Fluor-16β-methyl-17α-OCOR-3-oxo-androsta-4,9(11)-dien-17β-carbonsäure-methylester
(welcher z.B. durch Dehydratisieren eines entsprechenden
6α-Fluor-(11α oder 11β)-hydroxy-16β-methyl-17α-OCOR-3-oxo-androst-4-en-17β-carbonsäure-methylesters
mit Methansulfonylchlorid erhältlich ist) die Elemente der Hypochlorigen Säure in der unter Verfahrensvariante b) angegebenen Weise anlagert. (In diesen Zwischenprodukten hat R die eingangs angegebene Bedeutung).

Die erfindungsgemässe Veresterung (d.h. Umwandlung in den Methylester) gemäss der Verfahrensvariante d) kann in an sich bekannter Weise vorteilhaft so durchgeführt werden, dass man die freie Säure der Formel V mit Diazomethan behandelt. Die Reaktion wird in einem inerten organischen Lösungsmittel, wie einem aromatischen Kohlenwasserstoff, z.B. Benzol oder Toluol, einem Niederalkanol, z.B. vorzugsweise Methanol, einem Ether, z.B. Diethylether, 1,2-Dimethoxyethan, Tetrahydrofuran oder Dioxan, oder einem halogenierten Niederalkan, z.B. Dichlormethan oder Chloroform, oder einem Gemisch mehrerer solcher Lösungsmittel, bei einer Temperatur zwischen etwa –10 und etwa +30° durchgeführt, wobei vornehmlich Diazomethan entweder in gasförmigem Zustand (gegebenenfalls mittels eines inerten Treibgases, wie Argon oder Stickstoff) oder in Lösung, z.B. in einem der genannten Lösungsmittel, portionsweise einer Lösung der Säure (V) zugeführt wird. – Eine alternative Veresterungsmethode besteht im Behandeln der freien Säure in einem inerten organischen Lösungsmittel mit einem N,N′-disubstituierten O-Methylisoharnstoff, bei Temperaturen zwischen etwa 20° und etwa 100 °C. Man kann dabei auch das Reagens direkt im Reaktionsmedium bilden, indem man z.B. eine methanolische Lösung der freien Säure (V) mit einem N,N′-disubstituierten Carbodiimid, wie N,N′-Dicyclohexylcarbodiimid, behandelt. Ferner kann man auch konventionelle Veresterungsmethoden anwenden, wie Behandeln der freien Säure (V) mit überschüssigem Methanol unter Säurekatalyse (z.B. durch Schwefelsäure), Behandeln eines Alkalimetallsalzes, wie des Natrium-, Kalium- oder Lithiumsalzes, der Säure (V) mit einem Methylester einer starken Säure, z.B.

mit einem Methylhalogenid (wie Methyljodid oder Methylbromid), Dimethylsulfat oder einem organischen Sulfonat (wie Methyl-methansulfonat oder Methyl-p-toluolsulfonat), oder aber Behandeln eines gemischten Anhydrids der Säure V mit Methanol, gegebenenfalls in Anwesenheit einer Base, insbesondere einer tertiären organischen Base, wie eines tertiären Amins (z.B. Triethylamin oder N-Methylmorpholin) oder insbesondere des Pyridin oder eines seiner Homologen oder von Chinolin. Die zweite Säurekomponente des gemischten Anhydrids der Säure (V) kann entweder eine anorganische Säure, wie insbesondere Chlorwasserstoffsäure, sein (in welchem Falle das Chlorid der Säure V vorliegt), oder aber eine organische Säure, wie insbesondere Trifluoressigsäure oder auch eine Niederalkancarbonsäure, speziell eine der Formel RCOOH, worin R die eingangs angegebene Bedeutung hat. Derartige gemischte Anhydride der Säure (V) mit den genannten organischen Säuren können gegebenenfalls als Primärprodukte im Reaktionsgemisch vorliegen, welches z.B. beim Acylieren der
6α-Fluor-9α-chlor-11β,17α-dihydroxy-16β-methyl-3-oxo-androsta-1,4-dien-17β-carbonsäure
(VIA) (oder eines am 11β-Hydroxyl geschützten Derivats davon) mit einem Anhydrid der Formel $(R-CO)_2O$ (VIIA) oder $R-CO-O-CO-CF_3$ (VIIB), worin R die eingangs angegebene Bedeutung hat, analog Verfahrensvariante e) erhalten wird. Die erfindungsgemässe Veresterung gemäss Verfahrensvariante d) erfolgt dann anschliessend durch die Behandlung des rohen Reaktionsgemisches mit Methanol, welches vorzugsweise in einem solchen Überschuss angewendet wird, der zugleich auch das unverbrauchte Acylierungsreagens (VIIA bzw. VIIB) zu zersetzen vermag. Diese Veresterungsvariante kann man sowohl unter saurer wie auch basischer Katalyse durchführen.

Ausgangsstoffe der Formel V können in an sich bekannter Weise erhalten werden, z.B. durch den oxidativen Seitenketten-Abbau eines entsprechenden 21-Hydroxy-20-ketons der Pregna-Reihe, d.h. eines
6α-Fluor-9α-chlor-11β,21-dihydroxy-16β-methyl-17α-OCOR-pregna-1,4-dien-3,20
dions (IX), worin R die oben genannte Bedeutung hat. Die letztgenannten Verbindungen wiederum, sofern sie nicht bekannt sind, können in bekannter Weise ausgehend von
6α-Fluor-9α-chlor-11β,17α,21-trihydroxy-16β-methyl-pregna-1,4-dien-3,20-dion
(IXA) erhalten werden, indem man dieses mittels eines Niederalkyl-Orthoesters einer Carbonsäure R–COOH (VII) in den entsprechenden 17α,21-Orthoester des letztgenannten Triols (in welchem das dritte Sauerstoffatom dieses Orthoesters das ursprüngliche Niederalkyl trägt) umwandelt und diesen durch eine vorsichtige Hydrolyse gemäss einem herkömmlichen allgemeinen Verfahren in den gewünschten 17-Monoester überführt. – Der oxidative Abbau der Hydroxyacetyl-Seitenkette der Verbindungen der Formel (IX) erfolgt in an sich bekannter, methodisch optimierter Weise mit dazu geeigneten glykolspaltenden Oxidationsmitteln, wie insbesondere mit Perjodsäure oder ihren Salzen, vorteilhaft organischen Salzen, welche im Reaktionsgemisch durch Zugabe einer Base, wie Pyridin, gebildet werden. Die Umsetzung erfolgt zweckmässig in Gegenwart von Wasser in einem mit Wasser mischbaren organischen Lösungsmittel, wie einem Niederalkanol, z.B. Methanol, Ethanol, tert-Butylalkohol oder Ethylenglykol-mono-(methyl oder ethyl)-ether, oder einem cyclischen Ether, z.B. Tetrahydrofuran. Ebenso kann man auch mit einem Derivat des Wismutpentoxids, wie einem Alkalimetall-, z.B. Natrium- oder Kalium-, -wismutat, vorzugsweise in Essigsäure und insbesondere in wässriger Essigsäure, oxidieren.

Diesen oxidativen Abbau der Hydroxyacetyl-Seitenkette kann man auch für die Herstellung von Ausgangsstoffen anderer Verfahrensvarianten (z.B. der a, b, c und e) anwenden, indem man jeweils in einem im Ring A oder C geeignet substituierten, der Verbindung der Formel (IX) analogen 21-Hydroxy-20-oxo-Derivat die Seitenkette spaltet und die erhaltene freie 17β-Carbonsäure unter den oben geschilderten Bedingungen der erfindungsgemässen Verfahrensvariante d) in den Methylester umwandelt. Besonders zu erwähnen sind z.B. Derivate, die sich im Ring C durch die 9β,11β-Oxidogruppe, 9(11)-Doppelbindung oder geschützte 11β-Hydroxylgruppe und/oder im Ring A durch die gesättigte 1,2-Bindung von Pregnan-Verbindungen der Formel (IX) unterscheiden. – Mit Perjodsäure oder ihren Salzen kann man in analoger Weise auch das Triol (IXA) oder ein am 11β-Hydroxyl geschütztes Analoges davon zur
6α-Fluor-9α-chlor-11β,17α-dihydroxy-16β-methyl-3-oxo-androsta-1,4-dien-17β-carbonsäure
(VIA) bzw. ihrem 11-O-geschützter Derivat oxidieren, welche als Ausgangsstoffe oder Zwischenprodukte in Verfahrensvarianten e) bzw. f) Anwendung finden können.

Das erfindungsgemässe Acylieren des 17α-Hydroxyls gemäss Verfahrensvariante e) geschieht in an sich bekannter Weise durch Umsetzen des Esters VI mit der betreffenden Säure R–COOH (VII) oder vorzugsweise mit einem funktionellen Derivat davon, wie einem Halogenid, z.B. Chlorid, oder insbesondere einem Anhydrid, z.B. einem symmetrischen Anhydrid der Formel $(R-CO)_2O$ (VIIA) oder einem gemischten Anhydrid vom Typ $R-CO-O-COCF_3$ (VIIB), wobei R die obgenannte Bedeutung hat. Man kann auch in Gegenwart eines sauren Katalysators, wie p-Toluolsulfonsäure, Sulfosalicylsäure, Perchlorsäure oder eines sauren Ionenaustauschers vom Typ Amberlite[R] IR 120, acylieren; als Reaktionsmedium dient dabei üblicherweise ein cyclischer Kohlenwasserstoff, wie Benzol oder Toluol, oder ein chlorierter aliphatischer Kohlenwasserstoff, wie Methylenchlorid oder Chloroform. Zweckmässig arbeitet man mit überschüssigem Anhydrid bei Temperaturen zwischen etwa 20° bis etwa 100°C. Vornehmlich acyliert man unter basischer Katalyse, insbesondere in Gegenwart einer starken organischen Base, wie vorzugsweise 4-Dimethylamino-pyridin; als Acylierungsmittel verwendet man zweckmässig ein symmetrisches Anhydrid

(VIIA) und als Lösungsmittel bzw. Verdünnungsmittel z. B. eine heteroaromatische Base, wie Chinolin oder Pyridin oder vorzugsweise dessen Homologe, sowie auch cyclische Ether, wie Tetrahydrofuran, oder chlorierte aliphatische Kohlenwasserstoffe, wie Chloroform oder Methylenchlorid; die Reaktionstemperatur liegt üblicherweise zwischen etwa 0° bis etwa 50 °C, zweckmässig bei Zimmertemperatur oder leicht darunter.

Der Ausgangsstoff der Formel VI ist erhältlich z. B. durch herkömmliches Methylieren (z. B. durch die Veresterung gemäss der Verfahrensvariante d) der freien Säure (VIA), welche durch die oben beschriebene Oxidation aus dem Triol (IXA) der Pregnan-Reihe zugänglich ist.

Das erfindungsgemässe Abspalten einer Schutzgruppe vom 11β-Hydroxyl gemäss Verfahrensvariante f) erfolgt in an sich bekannter Weise unter Berücksichtigung der spezifischen Belange der jeweiligen Schutzgruppe. Zum Schutz der 11β-Hydroxylgruppe im Zusammenhang mit der vorliegenden Erfindung eignen sich z. B. leicht abspaltbare Estergruppen (Acylgruppen), wie Formyl, Chloracetyl und in erster Linie Trifluoracetyl, oder, in erster Linie, leicht abspaltbare Ethergruppen, insbesondere eine Tri(alkyl oder aryl)silyl-, vorzugsweise Tri(niederalkyl)silyl-, wie die Trimethylsilyl- oder die tert-Butyldimethylsilylgruppe.

Bekanntlich erfolgt die Abspaltung der Estergruppen besonders leicht unter solvolytischen, z. B. alkoholytischen oder hydrolytischen, Bedingungen mit milden basischen Mitteln, wie mit Alkalimetall- oder Erdalkalimetall-salzen schwacher anorganischer Säuren (z. B. mit Hydrocarbonaten, insbesondere Natrium- oder Kaliumhydrocarbonat) oder organischer Carbonsäuren (z. B. mit Formiaten oder Acetaten, insbesondere Kaliumformiat oder Kaliumacetat), oder aber mit heteroaromatischen Basen, z. B. Pyridin oder Collidin, in einem geeigneten, z. B. alkoholischen, wie methanolischen, oder wässrig-alkoholischen Medium. Eine bevorzugte Ausführungsart der Solvolyse der 11-Trifluoracetyloxygruppe ist z. B. in der Deutschen Patentschrift 1 593 519 beschrieben, bei welcher die veresterte Hydroxygruppe in 17α-Stellung unversehrt bleibt; man geht dabei so vor, dass man die 11β-Trifluor-acetyloxy-Verbindung in einem Niederalkanol mit dem Salz einer Säure, deren $pK_a$-Wert im Bereich von etwa 2,3 bis etwa 7,3 liegt, wie mit einem Alkalimetallazid (z. B. Natrium- oder Kaliumazid) oder Alkalimetallformiat (z. B. Natrium- oder Kaliumformiat) behandelt, wobei dieses Salz nur in katalytischen Mengen vorliegen kann. Ferner kann man die 11β-Trifluoracetylgruppe auch durch Behandeln mit Silikagel gemäss dem in der Deutschen OLS 2 144 405 beschriebenen Verfahren abspalten.

Diese leicht abspaltbaren Estergruppen werden zweckmässig schon in früheren Vorstufen der Synthese in die Ausgangsstoffe, insbesondere in Verbindungen der Pregnan-Reihe vor dem oxidativen Abbau der Hydroxyacetyl-Seitenkette, eingeführt; die Einführung wird in an sich bekannter Weise, insbesondere durch Acylieren mit einem entsprechenden Anhydrid, wie Trifluoressiganhydrid, Chloressiganhydrid oder gemischtem Anhydrid der Ameisensäure und Essigsäure, vorzugsweise in einer heteroaromatischen Base, wie Pyridin, und vorteilhaft bei einer Temperatur, welche die Raumtemperatur nicht übersteigt, vorgenommen.

Die erfindungsgemässe Abspaltung der 11-O-Trialkylsilyl-gruppen erfolgt auch in an sich bekannter Weise, z. B. durch Hydrolyse unter Katalyse mit schwach sauren Mitteln und Bedingungen, unter welchen die 17α-ständige veresterte Hydroxylgruppe, sowie die 17β-ständige Methoxycarbonylgruppe unversehrt bleiben, z. B. durch Einwirkung von Trifluoressigsäure in Gegenwart von Wasser in einem mit Wasser mischbaren organischen Lösungsmittel, wie einem Niederalkanol, z. B. Methanol, Ethanol, Isopropylalkohol oder tert-Butylalkohol, oder einem Ether, z. B. 2-(Methoxy- oder Ethoxy)-ethanol, Tetrahydrofuran oder Dioxan, ferner auch durch die Einwirkung wässriger Oxalsäure in diesen Lösungsmitteln oder aber wässriger Ameisen- oder Essigsäure allein, bei Temperaturen von etwa 0–50°, vorzugsweise in der Nähe der Raumtemperatur. – Vornehmlich kann diese Abspaltung auch mit Fluorid-Ionen, bzw. mit Fluorid-Ionen-abgebenden Mitteln, mit hoher Selektivität erfolgen, beispielsweise mit Fluoriden quaternärer Basen, wie insbesondere Tetraethylammoniumfluorid gemäss einem herkömmlichen allgemeinen Verfahren; als Lösungsmittel können z. B. die oben erwähnten Niederalkanole und Ether, aber auch Dimethylformamid und heteroaromatische Basen vom Typ Pyridin dienen; die Reaktionstemperatur liegt üblicherweise bei etwa 0–50 °C, vornehmlich im Bereich der Raumtemperatur.

Zweckmässig wird das 11β-Hydroxyl durch eine Trialkylsilylgruppe in einem früheren Synthesestadium geschützt, um Umwandlungen vornehmen zu können, welche gegebenenfalls das freie 11β-Hydroxyl beeinträchtigen könnten, wie z. B. die Acylierung des 17α-Hydroxyls, insbesondere unter energischeren Bedingungen. Die Einführung der Schutzgruppe erfolgt z. B. durch Behandeln einer geeigneten Verbindung mit freiem 11β-Hydroxyl mit einem Trialkylsilylhalogenid, wie insbesondere Trimethylsilylchlorid oder tert-Butyldimethylsilylchlorid, vorzugsweise in Gegenwart einer organischen Base, wie insbesondere Diethylamin oder Piperidin, und in einem Ether, z. B. Tetrahydrofuran, Dioxan, 1,2-Di-(methoxy oder ethoxy)-ethan oder Diethylether, oder einer heteroaromatischen Base, z. B. Pyridin oder dessen Homologen, als Reaktionsmedium bei Temperaturen von etwa 0–50 °C, vorzugsweise bei oder unterhalb der Raumtemperatur. Anschliessend an diese Einführung der Schutzgruppe können die gewünschten Umwandlungen erfolgen, wie insbesondere die Acylierung des 17α-Hydroxyls, aber auch die hydrolytische Freisetzung einer acylierten 21-Hydroxylgruppe, oxidativer Abbau der Hydroxyacetyl-Seitenkette, 1,2-Dehydrierung und/oder Veresterung des 17β-Carboxyls, die alle unter solchen Bedingungen durchführbar sind, welche die Schutzgruppe unversehrt lassen; bekanntlich ist diese Gruppe unter basischen Bedingungen besonders beständig.

Zum unmittelbaren Ausgangsstoff der vorliegenden Verfahrensvariante f), einem 11β-Trialkylsilyloxy-6α-fluor-9α-chlor-16β-methyl-17α-OCOR-3-oxo-androsta-1,4-dien-17β-carbonsäure-methylester (VIIIA), worin R die oben angegebene Bedeutung hat, kann man auf verschiedenen Wegen gelangen, z.B. in einer typischen Sequenz folgendermassen:

21-Acetoxy-6α-fluor-9α-chlor-11β,17α-dihydroxy-16β-methylpregna-1,4-dien-3,20-dion

wird mit einem Trialkylsilylchlorid unter basischen Bedingungen behandelt (wobei selektiv nur das 11β-Hydroxyl verethert wird), und im erhaltenen geschützten Zwischenprodukt wird nacheinander das acetylierte 21-Hydroxyl durch milde basische Hydrolyse freigesetzt, die Hydroxyacetyl-Seitenkette mit Perjodsäure gespalten, das freie 17α-Hydroxyl (vorzugsweise unter basischer Katalyse) acyliert und das 17β-Carboxyl (z.B. mit Diazomethan) verestert. Derselbe Ausgangsstoff (VIIIA) ist erhältlich auch auf einem anderen vorteilhaften Zugangswege, der zugleich eine ergänzende Alternative zur Verfahrensvariante e) darstellt, und zwar dadurch, dass man in

6α-Fluor-9α-chlor-11β,17α-dihydroxy-16β-methyl-3-oxo-androsta-1,4-dien-17β-carbonsäure-methylester

(VI) zunächst eine Trialkylsilyl-Schutzgruppe unter den oben geschilderten Bedingungen einführt, anschliessend gemäss Verfahrensvariante e) das 17α-Hydroxyl acyliert und zuletzt gemäss der vorliegenden Verfahrensvariante f) die Schutzgruppe abspaltet.

Die unmittelbaren Ausgangsstoffe für alle Verfahrensvarianten a–f, d.h. Verbindungen II bis VI und VIII, sind aus bekannten analog substituierten Vorstufen der Pregnan-Reihe nicht nur durch die oben spezifisch beschriebenen synthetischen Sequenzen, sondern auch durch andere zweckmässige Kombinationen des oxidativen Abbaus der Hydroxyacetyl-Seitenkette und der unter a–f beschriebenen allgemeinen Verfahren zugänglich. Bei allen diesen Umwandlungen, sowie bei den oben geschilderten erfindungsgemässen Verfahren als solchen, werden vorzugsweise Reaktionsmittel und Zwischenprodukte verwendet, die zu den besonders hervorgehobenen, insbesondere den spezifisch genannten, Endstoffen und Zwischenprodukten führen.

Sofern nicht spezifisch definiert wird, bezieht sich in der ganzen Beschreibung die Bezeichnung «nieder» im Zusammenhang mit einem Kohlenwasserstoffrest auf einen solchen mit höchstens 7 Kohlenstoffatomen.

Die Erfindung betrifft auch diejenigen Ausführungsformen der obigen Verfahren, bei denen man von einer auf irgendeiner Stufe als Zwischenprodukt erhältlichen Verbindung ausgeht und die fehlenden Schritte durchführt, oder bei denen ein Ausgangsstoff unter den Reaktionsbedingungen gebildet wird.

Die vorliegende Erfindung betrifft auch pharmazeutische Zusammensetzungen und Präparate für Menschen und Säugetiere, welche die neuen oben beschriebenen Verbindungen der Formel A in einer therapeutisch wirksamen Menge als aktive Substanz zusammen mit einem pharmazeutischen Trägermittel enthalten, sowie ihre Herstellung. Als Träger verwendet man organische oder anorganische Stoffe, die vor allem für die topische oder die Inhalationsverabreichung, z.B. in Form eines Aerosols, mikropulverisierten Pulvers oder einer feinversprühten Lösung, geeignet sind. Für die Bildung derselben kommen solche Stoffe in Frage, die mit den neuen Verbindungen nicht reagieren, wie z.B. Wasser, Gelatine, Milchzucker, Stärke, Magnesiumstearat, Talk, pflanzliche Öle, Benzylalkohol, Gummi, Polyalkylenglykole, Vaseline, Cholesterin und andere bekannte Arzneimittelträger. Die pharmazeutischen Präparate und Zusammensetzungen können insbesondere in flüssiger oder halbflüssiger Form als Lösungen, Suspensionen, Emulsionen, Salben oder Cremen vorliegen. Gegebenenfalls sind diese pharmazeutischen Präparate sterilisiert und/oder enthalten Hilfsstoffe, wie Konservierungs-, Stabilisierungs-, Netz- oder Emulgiermittel. Sie können auch noch andere therapeutisch wertvolle oder biologisch wirksame Stoffe enthalten.

Erfindungsgemäss kommen in erster Linie topisch anwendbare pharmazeutische Präparate, wie Cremen, Salben, Pasten, Schäume, Tinkturen und Lösungen, in Frage, die von etwa 0,001% bis etwa 0,5%, vorzugsweise etwa 0,005 bis etwa 0,05%, des Wirkstoffs enthalten.

Crèmen sind Öl-in-Wasser-Emulsionen, die mehr als 50% Wasser aufweisen. Als ölige Grundlage verwendet man in erster Linie Fettalkohole, z.B. Lauryl-, Cetyl- oder Stearylalkohol, Fettsäuren, z.B. Palmitin- oder Stearinsäure, flüssige bis feste Wachse, z.B. Isopropylmyristat, Wollwachs oder Bienenwachs, und/oder Kohlenwasserstoffe, z.B. Vaseline (Petrolatum) oder Paraffinöl. Als Emulgatoren kommen oberflächenaktive Substanzen mit vorwiegend hydrophilen Eigenschaften in Frage, wie entsprechende nichtionische Emulgatoren, z.B. Fettsäureester von Polyalkoholen oder Ethylenoxidaddukte davon, wie Polyglycerinfettsäureester oder Polyoxyethylensorbitan-fettsäureester (Tweens), ferner Polyoxyethylen-fettalkoholether oder -fettsäureester, oder entsprechende ionische Emulgatoren, wie Alkalimetallsalze von Fettalkoholsulfaten, z.B. Natriumlaurylsulfat, Natriumcetylsulfat oder Natriumstearylsulfat, die man üblicherweise in Gegenwart von Fettalkoholen, z.B. Cetylalkohol oder Stearylalkohol, verwendet. Zusätze von Wasserphase sind u.a. Mittel, welche die Austrocknung der Crème vermindern, z.B. Polyalkohole, wie Glycerin, Sorbit, Propylenglykol und/oder Polyethylenglykole, ferner Konservierungsmittel und Riechstoffe.

Salben sind Wasser-in-Öl-Emulsionen, die bis zu 70%, vorzugsweise jedoch von etwa 20% bis etwa 50% Wasser oder wässrige Phase enthalten. Als Fettphase kommen in erster Linie Kohlenwasserstoffe, z.B. Vaseline, Paraffinöl und/oder Hartparaffine in Frage, die zur Verbesserung des Wasserbindungsvermögens vorzugsweise geeignete

Hydroxyverbindungen, wie Fettalkohole oder Ester davon, z.B. Cetylalkohol oder Wollwachsalkohole, bzw. Wollwachs, enthalten. Emulgatoren sind entsprechende lipophile Substanzen, wie Sorbitan-fettsäureester (Spans), z.B. Sorbitanoleat und/oder Sorbitanisostearat. Zusätze zur Wasserphase sind u.a. Feuchthaltungsmittel, wie Polyalkohole, z.B. Glycerin, Propylenglykol, Sorbit und/oder Polyethylenglykol, sowie Konservierungsmittel und Riechstoffe.

Fettsalben sind wasserfrei und enthalten als Grundlage insbesondere Kohlenwasserstoffe, z.B. Paraffin, Vaseline und/oder flüssige Paraffine, ferner natürliche oder partial-synthetische Fette, z.B. Kokosfettsäuretriglycerid, oder vorzugsweise gehärtete Öle, z.B. hydriertes Erdnuss- oder Rizinusöl, ferner Fettsäurepartialester des Glycerins, z.B. Glycerinmono- und -distearat, sowie z.B. die im Zusammenhang mit den Salben erwähnten, die Wasseraufnahmefähigkeit steigernden Fettalkohole, Emulgatoren und/oder Zusätze.

Pasten sind Crèmen und Salben mit sekretabsorbierenden Puderbestandteilen, wie Metalloxiden (z.B. Titandioxid oder Zinkoxid), ferner Talk und/oder Aluminiumsilikaten, welche die Aufgabe haben, vorhandene Feuchtigkeit oder Sekrete zu binden.

Tinkturen und Lösungen weisen meistens eine wässrige-ethanolische Grundlage auf, der u.a. Polyalkohole, z.B. Glycerin, Glykole, und/oder Polyethylenglykol, als Feuchthaltemittel zur Herabsetzung der Verdunstung, und rückfettende Substanzen, wie Fettsäureester mit niedrigen Polyethylenglykolen, d.h. im wässerigen Gemisch lösliche, lipophile Substanzen als Ersatz für die der Haut mit dem Ethanol entzogenen Fettsubstanzen, und, falls notwendig, andere Hilfs- und Zusatzmittel beigegeben sind.

Als pharmazeutische Zusammensetzungen für die Verabreichung durch Inhalation von Aerosolen oder Sprays sind geeignet z.B. Lösungen, Suspensionen oder Emulsionen des erfindungsgemässen Wirkstoffes der Formel A mit einem geeigneten pharmazeutisch annehmbaren Lösungsmittel, wie insbesondere Ethanol und Wasser, oder einem Gemisch solcher Lösungsmittel. Sie können nach Bedarf auch andere pharmazeutische Hilfsstoffe, wie nicht-ionische oder anionische oberflächenaktive Mittel, Emulgatoren und Stabilisatoren, sowie Wirkstoffe anderer Art enthalten und vor allem zweckmässig mit einem Treibgas, wie einem inerten Gas unter erhöhtem Druck oder insbesondere mit einer leicht flüchtigen, vorzugsweise unter normalem atmosphärischem Druck unterhalb der üblichen Raumtemperatur (z.B. zwischen etwa –30 und +10 °C) siedenden Flüssigkeit, wie einem mindestens teilweise fluorierten polyhalogenierten Niederalkan, oder einem Gemisch solcher Flüssigkeiten, vermischt werden. Derartige pharmazeutische Zusammensetzungen, welche vorwiegend als Zwischenprodukte oder Vorratsgemische für die Herstellung der entsprechenden Arzneimittel in fertiger Form verwendet werden, enthalten den Wirkstoff üblicherweise in einer Konzentration von etwa 0,01 bis etwa 5, insbesondere von etwa 0,1 bis etwa 1 Gewichts-%. – Zur Herstellung von Arzneimitteln in fertiger Form wird eine solche pharmazeutische Zusammensetzung in geeignete Behälter, wie Flacons und Druckflaschen, abgefüllt, welche mit einer für solche Zwecke geeigneten Versprühungseinrichtung bzw. Ventil versehen sind. Das Ventil ist vorzugsweise als ein Dosierungsventil konstruiert, welches bei der Betätigung eine vorbestimmte Menge der Flüssigkeit, entsprechend einer vorbestimmten Dosis des Wirkstoffs, freigibt. Bei Herstellung der fertigen Arzneimittelform kann man auch so vorgehen, dass man entsprechende Mengen der als Vorratslösung vorliegenden pharmazeutischem Zusammensetzung und des Treibmittels separat in die Behälter abfüllt.

Schäume werden aus Druckbehältern verabreicht und sind in Aerosolform vorliegende flüssige Öl-in-Wasser-Emulsionen, wobei halogenierte Kohlenwasserstoffe, wie Chlorfluorniederalkane (z.B. Dichlordifluormethan und Dichlortetrafluorethan) als Treibmittel verwendet werden. Als Ölphase verwendet man u.a. Kohlenwasserstoffe, z.B. Paraffinöl, Fettalkohole, z.B. Cetylalkohol, Fettsäureester, z.B. Isopropylmyristat, und/oder andere Wachse. Als Emulgatoren verwendet man u.a. Gemische von solchen mit vorwiegend hydrophilen Eigenschaften, wie Polyoxyethylen-sorbitan-fettsäureester (Tweens), und solchen mit vorwiegend lipophilen Eigenschaften, wie Sorbitanfettsäureester (Spans). Dazu kommen die üblichen Zusätze, wie Konservierungsmittel.

Die Herstellung der topisch verwendbaren pharmazeutischen Zusammensetzungen und Präparate erfolgt in an sich bekannter Weise, z.B. durch Lösen oder Suspendieren des Wirkstoffs in der Grundlage oder in einem Teil davon, falls notwendig. Bei Verarbeitung des Wirkstoffs als Lösung wird dieser in der Regel vor der Emulgierung in einer der beiden Phasen gelöst; bei Verarbeitung als Suspension wird er nach der Emulgierung mit einem Teil der Grundlage vermischt und dann dem Rest der Formulierung beigegeben.

Die Dosierung des Wirkstoffs, z.B. der oben besonders hervorgehobenen Verbindungen, erfolgt im Prinzip analog derjenigen von anerkannten topischen Antiinflammatorika vom Corticoid-Typ, einschliesslich der Inhalationspräparate; sie hängt jedoch auch einerseits von Spezies, Körpergewicht, Alter und individuellem Zustand des Warmblüters, andererseits von der Applikationsweise ab; eine geeignete Dosis kann im Routine-Test in bekannter Weise für jeden individuellen Fall festgestellt werden.

Die Erfindung betrifft auch die Verwendung einer Verbindung der Formel A zur Herstellung von Mitteln zur Linderung oder Behebung von krankhaften entzündlichen Zuständen des Körpers, und insbesondere der Haut, eines Warmblüters, vor allem des Menschen, welche durch die Behandlung dieses Körpers oder Körperteils, vorzugsweise in topischer Applikation, mit einer antiinflammatorisch wirksamen Menge einer Verbindung der Formel A, allein oder in Form eines

pharmazeutischen Präparats, charakterisiert ist. – Unter der Bezeichnung «eine antiinflammatorisch wirksame Menge» ist eine solche Menge des Wirkstoffs zu verstehen, die zu einer signifikanten Inhibition der Entzündung ausreicht.

Ebenfalls betrifft die Erfindung die Verwendung einer Verbindung der Formel A zur Herstellung von Mitteln zur Linderung oder Behebung krankhafter allergischer Zustände und/oder Symptome des Körpers eines Säugers, insbesondere des Menschen, welche insbesondere bei Asthma vorkommen. Diese Anwendung bzw. die entsprechende Heilmethode, ist durch die Behandlung des leidenden Körpers bzw. Körperteils mit einer antiallergisch wirksamen Menge einer Verbindung der Formel A allein oder in Form eines Arzneimittels, insbesondere einer zur Inhalation bestimmten pharmazeutischen Zusammensetzung, charakterisiert. Unter der Bezeichnung «eine antiallergisch wirksame Menge» ist eine solche Menge des Wirkstoffes zu verstehen, die zu einer signifikanten Behebung der allergischen Reaktion, wie Bronchokontraktion, ausreicht.

In den nachfolgenden Beispielen wird die praktische Durchführung der vorliegenden Erfindung noch näher illustriert, ohne sie dadurch in ihrem Umfang einzuschränken. Die Temperaturen werden vor- und nachstehend in Celsiusgraden angegeben; wenn nicht spezifisch bezeichnet, sind Lösungsmittelgemische in Vol.% oder in Vol.:Vol-Verhältnis, Feststofflösungen in Gew.%, d.h. als Gewicht des Feststoffs (in g) in Volumenteilen (in ml) der Lösung angegeben.

Beispiel 1

Eine Lösung von 1,340 g
9β,11β-Epoxy-6α-fluor-17α-hydroxy-16β-methyl-3-oxo-androsta-1,4-dien-17β-carbonsäure-methylester-17-propionat
in 66 ml Chloroform wird bei gewöhnlicher Temperatur unter Rühren mit trockenem Chlorwasserstoff gesättigt. Nach 4-stündiger Einwirkung verdünnt man mit 200 ml Methylenchlorid und schüttelt nacheinander mit 100 ml frisch bereiteter 1M Ammoniumhydrogencarbonatlösung, 2 mal mit 40 ml 0,5M Ammoniumhydrogencarbonatlösung und 5 mal mit 40 ml Wasser aus. Die organischen Phasen werden vereinigt, mit Natriumsulfat getrocknet, filtriert und im Vakuum, zuletzt unter mehrmaligem Zusetzen von Ether, eingedampft. Man erhält ca. 1,5 g kristallines Rohprodukt, welches in 50 ml Methylenchlorid gelöst und an einer in Methylenchlorid bereiteten Säule von 250 g Kieselgel (0,063–0,200 mm, entaktiviert mit 10% Wasser) unter Verwendung von Methylenchlorid-Ethylacetat-Gemischen von steigendem Ethylacetat-Gehalt gereinigt wird. Aus den mit Methylenchlorid-Ethylacetat (95:5) eluierten, dünnschichtchromatographisch einheitlichen Anteilen erhält man durch Kristallisation aus Ether 1,12 g reines
9α-Chlor-6α-fluor-11β,17α-dihydroxy-16β-methyl-3-oxo-androsta-1,4-dien-17β-carbonsäure-methylester-17-propionat
vom F. 222–224°.

Das als Ausgangsmaterial verwendete
9β,11β-Epoxy-6α-fluor-17α-hydroxy-16β-methyl-3-oxo-androsta-1,4-dien-17β-carbonsäure-methylester-17-propionat
kann wie folgt hergestellt werden:

a) Eine Lösung von 19,50 g
9β,11β-Epoxy-6α-fluor-17α,21-dihydroxy-16β-methyl-pregna-1,4-dien-3,20-dion
[siehe C.A. 97 (11), 092636, Registry-No. 82662–44–0, erhältlich auch aus dem entsprechenden 17,21-Diacetat, siehe U.S. Patent 4 346 037 durch Hydrolyse mit Kaliumcarbonat in wässrigem Methanol] und 1,0 g p-Toluolsulfonsäure in 100 ml Dimethylformamid und 25 ml Orthopropionsäuretriethylester rührt man 4 Stunden bei 20–23°, versetzt die Lösung hierauf mit 8,2 ml Pyridin, verdünnt das Reaktionsgemisch mit 3000 ml Methylenchlorid und schüttelt mit 5 × 200 ml Wasser, unter Rückextraktion mit 5 × 400 ml Methylenchlorid, aus. Die gesammelte Methylenchloridlösung wird mit Natriumsulfat getrocknet, filtriert und eingedampft, wobei gegen den Schluss mehrfach Ether zugesetzt wird. Der nahezu farblose, kristalline Rückstand vom
9β,11β-Epoxy-6α-fluor-17α,21-dihydroxy-16β-methyl-pregna-1,4-dien-3,20-dion-17,21-(ethyl-orthopropionat)
wird ohne Reinigung weiterverarbeitet.

b) Das erhaltene Rohprodukt (23,4 g) wird in 3600 ml Ethanol aufgenommen, eine Lösung von 12,60 g Oxalsäure-dihydrat in 200 ml Ethanol und 200 ml Wasser zugesetzt, und das Ganze 20 Stunden bei 20–23° gerührt. Hierauf kühlt man die Reaktionslösung auf 0–3° ab und tropft unter Rühren 1700–1750 ml 0,1N Kaliumhydrogencarbonat-Lösung zu, bis der pH-Wert 5,0–5,5 beträgt. Man engt das Reaktionsgut alsdann im Vakuum auf ein Restvolumen von ca. 1000 ml ein und nimmt das ausgeschiedene Hydrolyseprodukt in total 3600 ml Methylenchlorid auf. Der mit total 1200 ml 0,1N Ammoniumhydrogencarbonat-Lösung und 1200 ml Wasser gewaschene Extrakt wird mit Natriumsulfat getrocknet, filtriert und eingedampft. Das erhaltene Rohprodukt wird zur Abtrennung des mitentstandenen 21-Monopropionats in 62,5 ml Methylenchlorid gelöst und nach Aufziehen auf eine in Methylenchlorid bereitete Säule von 2500 g Kieselgel (0,063–0,200 mm, entaktiviert mit 10% Wasser) chromatographiert. Durch Eluieren mit je 3750 ml Toluol-Ethylacetat-Gemisch (75:25), (70:30), (65:35) und (60:40) werden insgesamt 19,3 g der stärker polaren Komponente erhalten. Durch Umkristallisieren aus Ether, unter Verwendung von Methylenchlorid als Lösungsvermittler, erhält man 18,15 g reines
9β,11β-Epoxy-6α-fluor-17α,21-dihydroxy-16β-methyl-pregna-1,4-dien-3,20-dion-17-propionat
vom F. 180–182°.

c) Eine Lösung von 4,465 g
9β,11β-Epoxy-6α-fluor-17α,21-dihydroxy-16β-methyl-pregna-1,4-dien-3,20-dion-17-propionat
in 160 ml Methanol werden mit 40 ml gesättigter methanolischer Kupfer(II)-acetat-Lösung versetzt und unter Rühren Sauerstoff übergeleitet. Nach 2¼ Stunden gibt man 80 ml 0,05M Lösung des

Di-Natriumsalzes der Ethylendiamin-tetraessigsäure in Wasser zu und engt das Ganze im Vakuum auf ein Restvolumen von ca. 80 ml ein. Unter Zusetzen von insgesamt 180 ml Wasser wird das Methanol im Vakuum bei 45–50° Badtemperatur unter schliesslichem Konzentrieren auf ca. 50 ml restlos entfernt. Nach Stehen bei 0° und Zerstossen mit einem Glasstab wird das kristalline Produkt auf einer Nutsche gesammelt, mit eiskaltem Wasser ausgewaschen und bei gewöhnlicher Temperatur über Calciumchlorid getrocknet. Man erhält so 4,60 g
9β,11β-Epoxy-6α-fluor-17α-hydroxy-16β-methyl-3,20-dioxo-pregna-1,4-dien-21-al-17-propionat
vom F. 94–96°.

d) 4,60 g
9β,11β-Epoxy-6α-fluor-17α-hydroxy-16β-methyl-3,20-dioxo-pregna-1,4-dien-21-al-17-propionat
übergiesst man mit 100 ml Methylenchlorid und gibt unter Feuchtigkeitsausschluss in Argonatmosphäre 10 g 3-Chlor-perbenzoesäure hinzu. Nach 8-stündigem Rühren bei gewöhnlicher Temperatur ist mittels Dünnschichtchromatographie kein Edukt mehr nachweisbar. Die gelbe Lösung wird mit 400 ml Ether und 1500 ml Methylenchlorid-Ether (1:4) verdünnt, mit ca. 100 g Eis versetzt, und hierauf das überschüssige Oxydationsmittel durch Schütteln mit 600 ml eiskalter 0,2M Natriumjodidlösung, 120 ml 1N Essigsäure und 600 ml 0,2M Natriumthiosulfatlösung zerstört. Anschliessend wird mit gesättigter Natriumsulfatlösung gewaschen. Die wässrigen Auszüge werden mit Methylenchlorid-Ether (1:4)-Gemisch nachextrahiert und die organische Phase wird mit der Hauptlösung vereinigt. Nach Trocknen mit Natriumsulfat und Filtrieren engt man auf ein Restvolumen von ca. 400 ml ein, kühlt das Konzentrat auf 0–3° ab und versetzt mit eiskalter ca. 0,5M ätherischer Diazomethanlösung (total ca. 140 ml), bis die blasse Gelbfärbung während 30 Minuten bestehen bleibt. Beim Eindampfen unter vermindertem Druck erhält man einen öligen Rückstand, der in 125 ml Toluol gelöst und an einer in Toluol bereiteten Säule von 500 g Kieselgel (0,063–0,200 mm, entaktiviert mit 10% Wasser) chromatographiert wird. Durch Eluieren mit Toluol-Ethylacetat (97,5:2,5)- und (90:10)-Gemisch und Kristallisation der mit Methylenchlorid-Ether-Gemischen gesammelten reinen Anteile aus Ether, erhält man total 2,70 g
9β,11β-Epoxy-6α-fluor-17α-hydroxy-16β-methyl-3-oxo-androsta-1,4-dien-17β-carbonsäure-methylester-17-propionat
von F. 203–205°. In analoger Weise können auch 17-Acetate und 17-Pivalate der unter a-e erwähnten Zwischenprodukte erhalten werden.

Beispiel 2

Zu einer Lösung von 3,00 g
9α-Chlor-6α-fluor-11β,17α-dihydroxy-16β-methyl-3-oxo-androsta-1,4-dien-17β-carbonsäure-17-propionat
[Rohprodukt enthaltend 3-Chlorbenzoesäure; siehe weiter unten] in 250 ml Ether gibt man bei 0–3° langsam 75 ml ca. 0,5M-Lösung von Diazomethan in Ether zu, wobei die gelbe Farbe zum Schluss bestehen bleibt. Nach einer weiteren halben Stunde wird die Lösung unter vermindertem Druck eingedampft, der ölige Rückstand in wenig Methylenchlorid-Toluol (1:1) gelöst und unter Nachspülen mit Toluol auf eine in Toluol bereitete Säule von 180 g Kieselgel 0,063–0,200 mm aufgezogen, und die Säule mit Toluol-Ethylacetat (4:1) eluiert. Nach einem kleinen Vorlauf, welcher 60 mg des in 6-Stellung epimeren
9α-Chlor-6β-fluor-11β,17α-dihydroxy-16β-methyl-3-oxo-androsta-1,4-dien-17β-carbonsäure-methylester-17-propionat,
F. 214–218° (aus Ether-Petrolether) enthält, folgen Fraktionen der Hauptkomponente. Durch Eindampfen und Kristallisieren des Rückstandes aus Methylenchlorid-Ether erhält man das gewünschte
9α-Chlor-6α-fluor-11β,17α-dihydroxy-16β-methyl-3-oxo-androsta-1,4-dien-17β-carbonsäuremethylester-17-propionat
vom F. 220–222°, welches mit dem Produkt des Beispiels 1 identisch ist.

Das als Ausgangsmaterial verwendete
9α-Chlor-6α-fluor-11β,17α-dihydroxy-16β-methyl-3-oxo-androsta-1,4-dien-17β-carbonsäure-17-propionat
kann wie folgt hergestellt werden:

a) 1,16 g
9β,11β-Epoxy-6α-fluor-17α-hydroxy-16β-methyl-3,20-dioxo-pregna-1,4-dien-21-al-17-propionat
(siehe Beispiel 1c) werden in trockener Argon-Atmosphäre in 25 ml Methylenchlorid gelöst und mit 2,5 g 3-Chlor-perbenzoesäure behandelt. Nach 8-stündigem Rühren bei gewöhnlicher Temperatur wird die Lösung mit 200 ml Ether und 50 ml Methylenchlorid verdünnt, unter Zugabe von Eis nacheinander mit 150 ml 0,2M-Natriumjodidlösung, 30 ml 1N Essigsäure und 150 ml 0,2M-Natriumthiosulfatlösung ausgeschüttelt und mit Natriumsulfat getrocknet. Durch Eindampfen wird ein kristalliner Rückstand erhalten, der neben 3-Chlorbenzoesäure im wesentlichen aus
9β,11β-Epoxy-6α-fluor-17α-hydroxy-16β-methyl-3-oxo-androsta-1,4-dien-17β-carbonsäure-17-propionat
besteht.

b) Auf die Oberfläche der Lösung von 3,4 g des obigen Produktgemisches in 300 ml Chloroform leitet man unter Kühlung auf 0–3° und unter Rühren trockenen Chlorwasserstoff. Die Reaktion wird dünnschichtchromatographisch auf Kieselgelplatten unter Verwendung von Chloroform-Methanol-Wasser (89:10:1) verfolgt und ist in der Regel nach ca. 5 Std. beendet. Das Reaktionsgemisch wird mit 100 ml Chloroform verdünnt, eiskalt mit 2N-Ammoniumhydrogencarbonatlösung auf pH von etwa 6 gebracht, mit Wasser gewaschen, mit Natriumsulfat getrocknet und eingedampft, gegen Schluss unter mehrfachem Zusetzen von Ether. Der erhaltene kristalline Rückstand besteht gemäss Dünnschichtanalyse zu mehr als 90% aus

9α-Chlor-6α-fluor-11β,17α-dihydroxy-16β-methyl-3-oxo-androsta-1,4-dien-17β-carbonsäure-17-propionat

neben 3-Chlorbenzoesäure aus der vorangehenden Stufe. Dieses Produkt kann ohne Reinigung in der Veresterungsstufe eingesetzt werden.

Beispiel 3

Zu einer gerührten Lösung von 15 mg p-Toluolsulfonsäure in 16,4 ml Propionsäure wird bei etwa 15° unter Feuchtigkeitsausschluss 3,6 ml Trifluoressigsäureanhydrid zugetropft und das Gemisch weitere 4 Stunden bei Raumtemperatur gerührt. Mit dieser frisch zubereiteten Reagenzlösung wird in trockener Argon-Atmosphäre 109 mg

9α-Chlor-6α-fluor-11β,17α-dihydroxy-16β-methyl-3-oxo-androsta-1,4-dien-17β-carbonsäure-methylester

übergossen und 4 Stunden bei Raumtemperatur gerührt. (Dabei geht das Ausgangsmaterial allmählich in Lösung und das Produkt beginnt sich kristallin auszuscheiden). Das Reaktionsgemisch wird mit 80 ml Ethylacetat verdünnt und eiskalt nacheinander mit 100 ml Wasser, 30 ml 1,25M-Natriumhydrogencarbonatlösung und 100 ml Wasser gewaschen, und die wässrigen Anteile werden zweimal mit je 50 ml Ethylacetat nachextrahiert. Die vereinigten organischen Auszüge werden über Natriumsulfat getrocknet und in Vakuum eingedampft, und der Rückstand wird in etwa 10 ml Methylenchlorid gelöst und auf eine Säule von 25 g Kieselgel aufgetragen. Durch Eluieren mit Methylenchlorid-Ethylacetat (4:1) erhaltene Fraktionen ergeben nach Entfernung der Lösungsmittel und Umkristallisieren aus Ether-Petrolether das gewünschte

9α-Chlor-6α-fluor-11β,17α-dihydroxy-16β-methyl-3-oxo-androsta-1,4-dien-17β-carbonsäure-methylester-17-propionat,

F. 222–223°, welches mit dem Produkt des Beispiels 1 identisch ist.

Der Ausgangsstoff kann wie folgt hergestellt werden:

a) Eine Lösung von 9,76 g

9β,11β-Epoxy-6α-fluor-17α,21-dihydroxy-16β-methyl-pregna-1,4-dien-3,20-dion

in 1000 ml Tetrahydrofuran versetzt man unter Rühren mit einem Oxidationsreagenz, welches durch Lösen von 51,7 g Perjodsäure-dihydrat in 150 ml Wasser und 20 ml Pyridin und Auffüllen mit Wasser auf ein Endvolumen von total 225 ml separat bereitet wird. Nach 17-stündigem Rühren bei Raumtemperatur wird das Reaktionsgemisch mit 600 ml Wasser verdünnt und im Wasserstrahlvakuum auf etwa 400 ml eingeengt. Der ausgefallene Feststoff wird auf einer Nutsche gesammelt, mit eiskaltem Wasser ausgewaschen und im Hochvakuum bei 20° getrocknet. Die erhaltene kristalline

9β,11β-Epoxy-6α-fluor-17α-hydroxy-16β-methyl-androsta-1,4-dien-17β-carbonsäure

kann ohne zusätzliche Reinigung weiterverarbeitet werden.

b) Eine Lösung von 5,80 g dieser Säure in 500 ml Methanol versetzt man portionsweise bei 0–3° mit insgesamt 350 ml ca. 0,6M-Lösung von Diazomethan in Ether, bis die Lösung gelb gefärbt bleibt. Nach weiterem einstündigen Rühren bei 0–3° wird die Reaktionslösung im Wasserstrahlvakuum stark eingeengt, das Kristallisat in wenig Ether aufgeschlämmt und genutscht. Durch Trocknen erhält man

9β,11β-Epoxy-6α-fluor-17α-hydroxy-16β-methyl-androsta-1,4-dien-17β-carbonsäure-methylester

in schwach beige-stichigen Kristallen, F. 207–209°.

c) 5,20 g dieser Verbindung werden in Argon-Atmosphäre unter Rühren in 400 ml Chloroform gelöst, und auf die Oberfläche der Lösung wird bei 0–3° trockener Chlorwasserstoff unter Rühren aufgeleitet. Der Fortschritt der Reaktion wird dünnschichtchromatographisch [Kieselgelplatte; Fliessmittel: Toluol-Ethylacetat (1:9)] verfolgt, bis die Umsetzung nach etwa 3–3,5 Stunden beendet ist. Das Reaktionsgemisch wäscht man nacheinander zweimal mit je 400 ml eiskaltem Wasser, 200 ml 1,25M-Natriumhydrogencarbonatlösung und zweimal je 300 ml eiskaltem Wasser, und die wässrigen Auszüge werden auch mit je 200 ml Chloroform nachextrahiert. Die vereinigte organische Phase wird über Natriumsulfat getrocknet, auf ca. 300 ml eingeengt und unter Nachspülen mit Methylenchlorid auf eine Säule von 700 ml Kieselgel aufgetragen. Durch Elution mit Toluol-Ethylacetat (1:3), konventionelle Aufarbeitung der Fraktionen und Umkristallisieren aus Hexan-Ether wird

9α-Chlor-6α-fluor-11β,17α-dihydroxy-16β-methyl-3-oxo-androsta-1,4-dien-17β-carbonsäure-methylester

erhalten, F. 228–229° (nach Umwandlung der Kristallform bei 199–203°).

Beispiel 4

Eine unter Argon bereitete und auf −10 bis −12° gekühlte Lösung von 107,7 mg

6α-Fluor-17α-hydroxy-16β-methyl-3-oxo-androsta-1,4,9(11)-trien-17β-carbonsäure-methylester-17-propionat

in 2,3 ml Aceton wird nacheinander mit 0,25 ml 0,5N wässriger Perchlorsäure und tropfenweise mit 0,13 ml tert-Butylhypochlorit unter Rühren versetzt, 1 Stunde bei 0–3° gerührt, in ein eiskaltes Gemisch von 0,30 ml 0,5M-Natriumhydrogencarbonatlösung und 20 ml Wasser gegossen und bei etwa 2,5 mbar auf ca. 5 ml eingeengt. Das Reaktionsprodukt wird in ein Gemisch von Methylenchlorid-Ether (1:2) aufgenommen, die Lösung eiskalt mit 10 ml einer (0,1M-Natriumjodid + 0,1N-Schwefelsäure)-Lösung, mit 10 ml 0,1N-Natriumthiosulfat, zweimal mit je 10 ml Ammoniumhydrogencarbonatlösung und mit Wasser gewaschen, und die wässrigen Auszüge mit demselben Lösungsmittelgemisch nachextrahiert. Die gesammelte organische Phase wird über Natriumsulfat getrocknet und unter vermindertem Druck eingeengt. Durch Umkristallisieren aus Methylenchlorid-Ether erhält man

9α-Chlor-6α-fluor-11β,17α-dihydroxy-16β-methyl-3-oxo-androsta-1,4-dien-17β-carbonsäure-methylester-17-propionat,
F. 219–222°, welches mit dem Produkt des Beispiels 1 identisch ist.

Der Ausgangsstoff kann beispielsweise wie folgt hergestellt werden:

Eine Lösung von 1,90 g
9β,11β-Epoxy-6α-fluor-17α-hydroxy-16β-methyl-3-oxo-androsta-1,4-dien-17β-carbonsäure-methylester-17-propionat
[siehe Beispiel 1d)] in 75 ml Tetrahydrofuran und 8,5 ml 65-proz. Jodwasserstoffsäure wird 12 Std. bei 40–43° gerührt, auf ca. 5° abgekühlt und mit 500 ml Ethylacetat verdünnt. Zur Entfernung des ausgeschiedenen Jods wird das Reaktionsgemisch mit einem eiskalten Gemisch von 400 ml 0,4N-Natriumthiosulfatlösung und 80 ml 2N Essigsäure ausgeschüttelt und danach noch mit 400 ml eiskaltem Wasser, 160 ml eiskalter 1,25M-Natriumhydrogencarbonatlösung und erneut mit 400 ml eiskaltem Wasser gewaschen, wobei die Auszüge der Reihe nach zweimal mit je 200 ml Ethylacetat nachextrahiert werden. Die vereinigte und mit Natriumsulfat getrocknete organische Phase wird im Vakuum eingedampft und das Rohprodukt auf einer Säule von 250 g Kieselgel chromatographiert; durch Eluieren mit Gemischen von Toluol-Ethylacetat (95:5) und (80:20), die konventionelle Aufarbeitung der Fraktionen und Umkristallisieren aus Ether-Petrolether gewinnt man das gewünschte
6α-Fluor-17α-hydroxy-16β-methyl-3-oxo-androsta-1,4,9(11)-trien-17β-carbonsäure-methylester-17-propionat, F. 164–165°.

Beispiel 5

Eine Lösung von 607 mg
9α-Chlor-6α-fluor-11β,17α-dihydroxy-16β-methyl-3-oxo-androst-4-en-17β-carbonsäure-methylester-17-propionat
und 1,58 g
2,3-Dichlor-5,6-dicyan-1,4-benzochinon
in 15 ml peroxidfreiem Dioxan wird in einem Druckgefäss unter Argon unter Rühren während 60 Std. auf 100° erwärmt. Nach Abkühlen wird das Gemisch in 125 ml Wasser gegossen, mit ca. 35 ml Dioxan nachgespült und durch Einengen im Hochvakuum bei 27–30° Badtemperatur auf ein Restvolumen von ca. 20 ml vom Dioxan befreit. Das Gemisch wird in 100 ml Wasser aufgeschwämmt und nacheinander mit 250, 100 und 100 ml eines Gemisches von Methylenchlorid-Ether (1:2) extrahiert. Die Auszüge werden dreimal mit je 50 ml eiskalter 2N-Natriumcarbonatlösung, fünfmal mit je 50 ml Wasser gewaschen, die vereinigten organischen Lösungen mit Natriumsulfat getrocknet und im Vakuum eingedampft. Der Rückstand wird durch konventionelle präparative Schichtchromatographie an Kieselgel mit Cyclohexan-Ethylacetat (1:1) als Fliessmittel gereinigt und aus Ether kristallisiert. Es resultiert
9α-Chlor-6α-fluor-11β,17α-dihydroxy-16β-methyl-3-oxo-androsta-1,4-dien-17β-carbonsäure-methylester-17-propionat,
F. 221–224°; welches mit dem Produkt des Beispiels 1 identisch ist.

Der Ausgangsstoff kann in analoger Weise, wie für die entsprechenden 1,4-Diene im Beispiel 1 ausführlich beschrieben wird, ausgehend vom
9β,11β-Epoxy-6α-fluor-17α,21-dihydroxy-16β-methyl-pregn-4-en-3,20-dion
über die entsprechenden 1,2-gesättigten Zwischenprodukte hergestellt werden.

Beispiel 6

Zu einer Lösung von 17 mg p-Toluolsulfonsäure in 6,0 ml Eisessig wird bei 15° unter Rühren und Feuchtigkeitsausschluss 2,3 ml Trifluoressigsäureanhydrid zugetropft und das Gemisch über Nacht stehengelassen. Mit 5,0 ml dieser Reagenzlösung wird in trockener Argonatmosphäre eine Lösung von 533 mg
9α-Chlor-6α-fluor-11β,17α-dihydroxy-16β-methyl-3-oxo-androsta-1,4-dien-17β-carbonsäure-methylester
(für Herstellung siehe Beispiel 3a–c) in 5,0 ml Methylenchlorid versetzt und 3 Stunden bei Raumtemperatur gerührt. Das Reaktionsgemisch wird in 60 ml eiskaltes Wasser gegossen und mit zwei Portionen von je 80 ml Ethylacetat extrahiert. Diese Extrakte werden separat nacheinander mit 80 ml Wasser, zweimal mit je 50 ml 1,25M-Natriumhydrogencarbonatlösung und zweimal mit je 50 ml Wasser eiskalt gewaschen, vereinigt, mit Natriumsulfat getrocknet und im Vakuum eingedampft. Durch Kristallisieren des Rückstandes aus Aceton wird
9α-Chlor-6α-fluor-11β,17α-dihydroxy-16β-methyl-3-oxo-androsta-1,4-dien-17β-carbonsäure-methylester-17-acetat,
F. 242–243°, erhalten. Eine weitere Menge des Produkts kann durch konventionelle Chromatographie der Mutterlaugen, z.B. an Kieselgel mit Toluol-Ethylacetat (9:1) als Eluiermittel, gewonnen werden.

Beispiel 7

Zu einer Lösung von 17 mg p-Toluolsulfonsäure in 10,8 ml Valeriansäure wird bei 15° unter Rühren und Feuchtigkeitsausschluss 2,3 ml Trifluoressigsäureanhydrid zugetropft und das Gemisch 24 Stunden bei Raumtemperatur stehengelassen. Mit 5,0 ml dieser Reagenzlösung wird in trockener Argonatmosphäre eine Lösung von 533 mg
9α-Chlor-6α-fluor-11β,17α-dihydroxy-16β-methyl-3-oxo-androsta-1,4-dien-17β-carbonsäure-methylester
(für Herstellung siehe Beispiel 3a–c) in 5,0 ml Methylenchlorid versetzt und 3 Stunden bei Raumtemperatur gerührt. Das Reaktionsgemisch wird mit 125 ml Ethylacetat extrahiert, auf 0–3° abgekühlt und nacheinander dreimal mit je 50 ml 2N-Natriumcarbonatlösung und dreimal mit je 50 ml Wasser eiskalt gewaschen. Die wässrigen Auszüge werden mit 75 ml Ethylacetat nachextrahiert, und die vereinigten organischen Auszüge mit Natriumsulfat getrocknet und im Vakuum eingedampft. Durch Chromatographie an einer Säule von 130 g Kieselgel, Elution mit Toluol-Ethylacetat (2:1), konventionelle Aufarbeitung der Eluate und Kristallisation des Produkts aus Methylenchlorid-Ether wird
9α-Chlor-6α-fluor-11β,17α-dihydroxy-16β-methyl-3-oxo-androsta-1,4-dien-17β-carbonsäure-methylester-17-valerianat,
F. 201–202°, erhalten.

Beispiel 8

Eine Salbe, enthaltend 0,1%
6α-Fluor-9α-chlor-11β-hydroxy-16β-methyl-17α-propionyloxy-3-oxo-androsta-1,4-dien-17β-carbonsäure-methylester
kann wie folgt hergestellt werden:

| Zusammensetzung | |
|---|---|
| 6α-Fluor-9α-chlor-11β-hydroxy-16β-methyl-17α-propionyloxy-3-oxo-androsta-1,4-dien-17β-carbonsäure-methylester | 0,1% |
| Vaseline | 45,0% |
| Paraffinöl | 19,6% |
| Cetylalkohol | 5,0% |
| Bienenwachs | 5,0% |
| Sorbitan-sesquioleat | 5,0% |
| p-Hydroxybenzoesäureester | 0,2% |
| Riechstoff | 0,1% |
| Wasser | 20,0% |

Die Fettstoffe und Emulgatoren werden zusammengeschmolzen. Das Konservierungsmittel wird in Wasser gelöst, und die Lösung in die Fettschmelze bei erhöhter Temperatur einemulgiert. Nach dem Erkalten wird eine Suspension des Wirkstoffs in einem Teil der Fettschmelze in die Emulsion eingearbeitet und schliesslich Riechstoff zugegeben.

Beispiel 9

Eine treibmittelhaltige, feststoffaerosolbildende Inhalationslösung enthaltend 0,1 Gew.-% Wirkstoff (z.B. des 17-Propionats gemäss Beispiel 1).

| Zusammensetzung: | Gew.-% |
|---|---|
| Wirkstoff, mikronisiert | 0,1 |
| Sorbitantrioleat | 0,5 |
| Treibmittel A (Trichlortrifluorethan) | 4,4 |
| Treibmittel B (Dichlordifluormethan und 1,2-Dichlortetrafluorethan) | 15,0<br>80,0 |

Herstellung: Der Wirkstoff wird unter Feuchtigkeitsausschluss mit Hilfe eines üblichen Homogenisators unter Zusatz des Sorbitantrioleats im Trichlortrifluorethan gelöst und in einen mit Dosierventil versehenen Aerosolbehälter abgefüllt; dieser wird verschlossen und unter Druck mit dem Treibmittel B aufgefüllt.

**Patentansprüche für die Vertragsstaaten: BE, CH, DE, FR, GB, IT, LI, LU, NL, SE**

1. Verbindungen der Formel

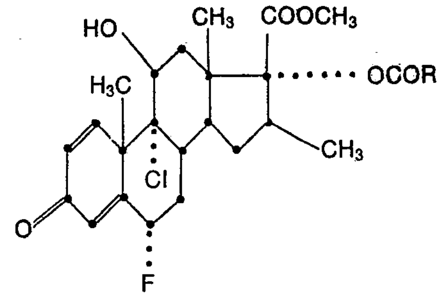

(A),

worin R ein Alkyl mit 1 bis 4 Kohlenstoffatomen bedeutet.

2. Eine Verbindung der Formel (A) gemäss Anspruch 1, worin R ein lineares Alkyl ist.

3. Eine Verbindung gemäss Anspruch 1, die der 9α-Chlor-6α-fluor-11β-hydroxy-16β-methyl-3-oxo-17α-propionyloxy-androsta-1,4-dien-17β-carbonsäure-methylester ist.

4. Eine Verbindung gemäss Anspruch 1, die der 9α-Chlor-6α-fluor-11β-hydroxy-16β-methyl-3-oxo-17α-acetoxy-androsta-1,4-dien-17β-carbonsäure-methylester ist.

5. Eine Verbindung gemäss Anspruch 1, die der 9α-Chlor-6α-fluor-11β-hydroxy-16β-methyl-3-oxo-17α-valeryloxy-androsta-1,4-dien-17β-carbonsäure-methylester ist.

6. Eine Verbindung gemäss einem der Ansprüche 1–5 zur therapeutischen Anwendung.

7. Eine Verbindung gemäss einem der Ansprüche 1–5 zur Behandlung von Hautkrankheiten und Asthma.

8. Verfahren zur Herstellung einer Verbindung der Formel (A) gemäss einem der Ansprüche 1–5, dadurch gekennzeichnet, dass man

a) eine 9β,11β-Oxidoverbindung der Formel

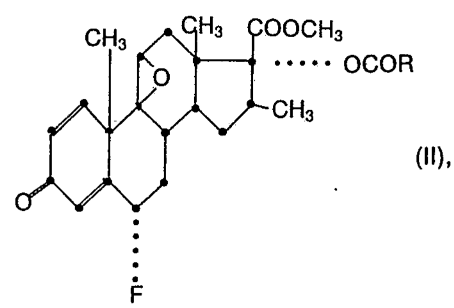

(II),

worin R die in einem der Ansprüche 1–5 genannte Bedeutung hat, mit Chlorwasserstoff oder einem Chlorwasserstoff-abgebenden Mittel behandelt, oder

b) an eine 9(11)-ungesättigte Verbindung der Formel

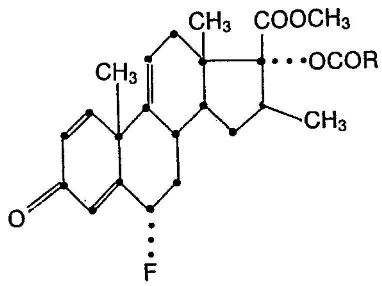

(III)

worin R die oben genannte Bedeutung hat, Elemente der Hypochlorigen Säure anlagert, oder

c) eine 1,2-gesättigte Verbindung der Formel

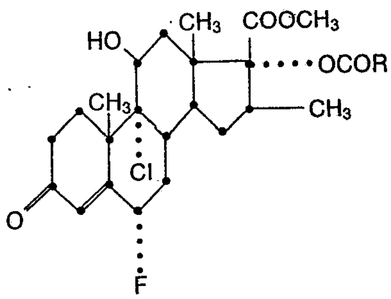

(IV)

worin R die oben genannte Bedeutung hat, in 1,2-Stellung dehydriert, oder

d) eine Carbonsäure der Formel

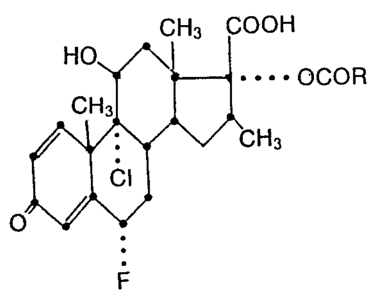

(V)

worin R die oben genannte Bedeutung hat, oder ein Salz oder ein reaktionsfähiges funktionelles Derivat davon, mit Hilfe eines methylierenden Mittels verestert, oder

e) den

9α-Chlor-6α-fluor-11β,17α-dihydroxy-16β-methyl-3-oxo-androsta-1,4-dien-17β-carbonsäure-methylester der Formel

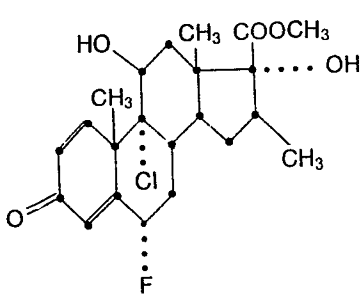

(VI)

mit einer Carbonsäure der Formel R-COOH (VII), worin R die oben genannte Bedeutung hat, oder einem reaktiven funktionellen Derivat davon, gegebenenfalls unter vorübergehendem Schutz des 11β-Hydroxyls, acyliert, oder

f) in einer Verbindung mit geschütztem 11β-Hydroxyl der Formel

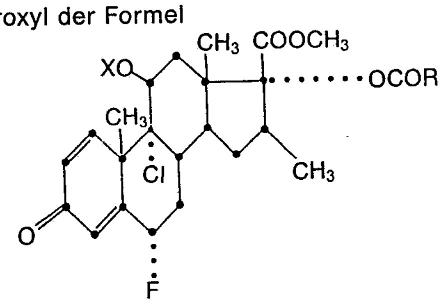

(VIII),

worin X eine Hydroxyl-Schutzgruppe darstellt und R die oben genannte Bedeutung hat, die Schutzgruppe X abspaltet.

9. Eine pharmazeutische Zusammensetzung enthaltend mindestens eine der in Ansprüchen 1–7 genannte Verbindung.

10. Verwendung einer Verbindung gemäss einem der Ansprüche 1–7 zur Herstellung eines Mittels zur Behandlung von Entzündungen oder Allergien.

**Patentansprüche für den Vertragsstaat: AT**

1. Verfahren zur Herstellung einer Verbindung der Formel

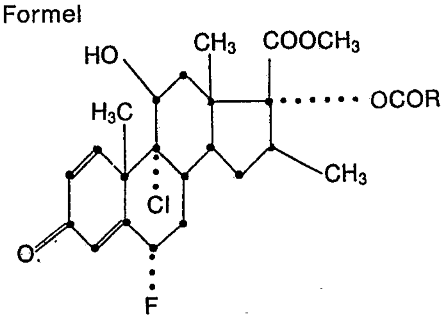

(A),

worin R ein Alkyl mit 1 bis 4 Kohlenstoffatomen bedeutet, dadurch gekennzeichnet, dass man

a) eine 9β,11β-Oxidoverbindung der Formel

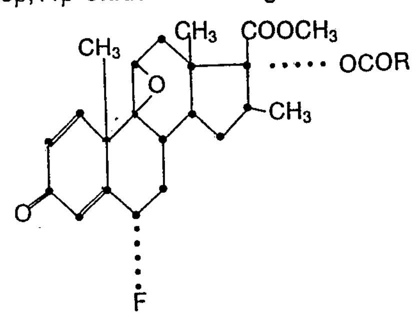

(II),

worin R die oben genannte Bedeutung hat, mit Chlorwasserstoff oder einem Chlorwasserstoff-abgebenden Mittel behandelt, oder

b) an eine 9(11)-ungesättigte Verbindung der Formel

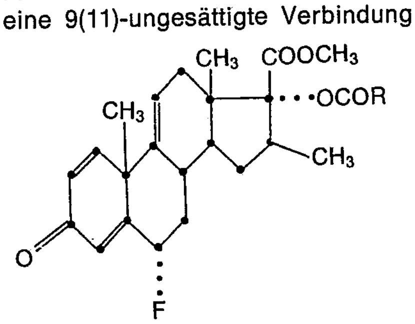

(III)

worin R die oben genannte Bedeutung hat, Elemente der Hypochlorigen Säure anlagert, oder

c) eine 1,2-gesättigte Verbindung der Formel

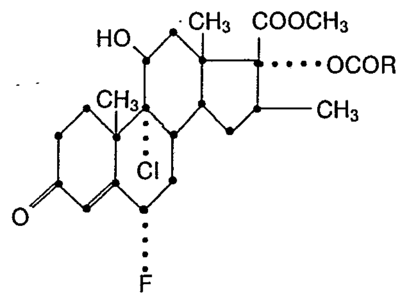

(IV)

worin R die oben genannte Bedeutung hat, in 1,2-Stellung dehydriert, oder

d) eine Carbonsäure der Formel

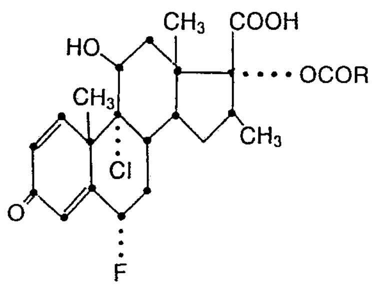

(V)

worin R die oben genannte Bedeutung hat, oder ein Salz oder ein reaktionsfähiges funktionelles Derivat davon, mit Hilfe eines methylierenden Mittels verestert, oder

e) den

9α-Chlor-6α-fluor-11β,17α-dihydroxy-16β-methyl-3-oxo-androsta-1,4-dien-17β-carbonsäure-methylester

der Formel

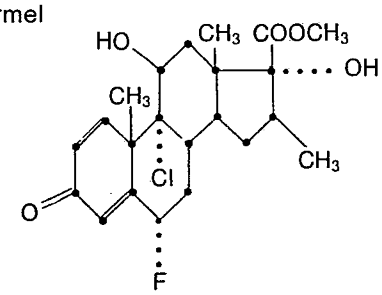

(VI)

mit einer Carbonsäure der Formel R-COOH (VII), worin R die oben genannte Bedeutung hat, oder einem reaktiven funktionellen Derivat davon, gegebenenfalls unter vorübergehendem Schutz des 11β-Hydroxyls, acyliert, oder

f) in einer Verbindung mit geschütztem 11β-Hydroxyl der Formel

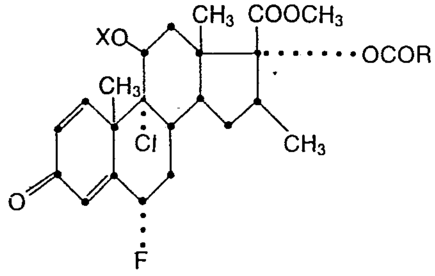

(VIII),

worin X eine Hydroxyl-Schutzgruppe darstellt und R die oben genannte Bedeutung hat, die Schutzgruppe X abspaltet.

2. Verfahren gemäss Anspruch 1, dadurch gekennzeichnet, dass man eine Verbindung der Formel A, worin R ein lineares Alkyl ist, herstellt.

3. Verfahren gemäss Anspruch, dadurch gekennzeichnet, dass man

9α-Chlor-6α-fluor-11β-hydroxy-16β-methyl-3-oxo-17α-propionyloxy-androsta-1,4-dien-17β-carbonsäure-methylester

herstellt.

4. Verfahren gemäss Anspruch 1, dadurch gekennzeichnet, dass man

9α-Chlor-6α-fluor-11β-hydroxy-16β-methyl-3-oxo-17α-acetoxy-androsta-1,4-dien-17β-carbonsäure-methylester

herstellt.

5. Verfahren gemäss Anspruch 1, dadurch gekennzeichnet, dass man

9α-Chlor-6α-fluor-11β-hydroxy-16β-methyl-3-oxo-17α-valeryloxy-androsta-1,4-dien-17β-carbonsäure-methylester

herstellt.

6. Verfahren zur Herstellung von pharmazeutischen Zusammensetzungen enthaltend mindestens eine der in Ansprüchen 1–5 genannte Verbindung, dadurch gekennzeichnet, dass man eine solche Verbindung mit mindestens einem pharmazeutisch annehmbaren Hilfsstoff vermischt.

**Claims for the Contracting States: BE, CH, DE, FR, GB, IT, LI, LU, NL, SE**

1. Compounds of the formula

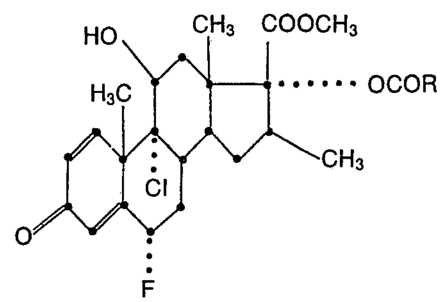

(A)

in which R represents alkyl having from 1 to 4 carbon atoms.

2. A compound of the formula (A) according to claim 1 in which R is a linear alkyl radical.

3. A compound according to claim 1 which is 9α-chloro-6α-fluoro-11β-hydroxy-16β-methyl-3-oxo-17α-propionyloxy-androsta-1,4-diene-17β-carboxylic acid methyl ester.

4. A compound according to claim 1 which is 9α-chloro-6α-fluoro-11β-hydroxy-16β-methyl-3-oxo-17α-acetoxy-androsta-1,4-diene-17β-carboxylic acid methyl ester.

5. A compound according to claim 1 which is 9α-chloro-6α-fluoro-11β-hydroxy-16β-methyl-3-oxo-17α-valeryloxy-androsta-1,4-diene-17β-carboxylic acid methyl ester.

6. A compound according to any one of claims 1 to 5 for therapeutic use.

7. A compound according to any one of claims 1 to 5 for treating skin disorders and asthma.

8. Process for the manufacture of a compound of the formula (A) according to any one of claims 1 to 5, characterised in that

a) a 9β,11β-oxido compound of the formula

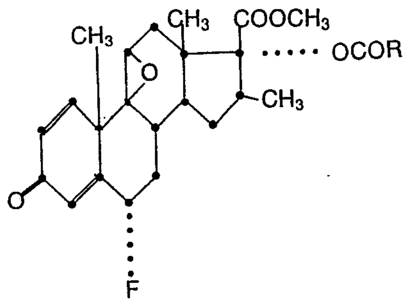

(II)

in which R has the meaning given in any one of claims 1 to 5 is treated with hydrogen chloride or with an agent yielding hydrogen chloride, or

b) elements of hypochlorous acid are added to a 9(11)-unsaturated compound of the formula

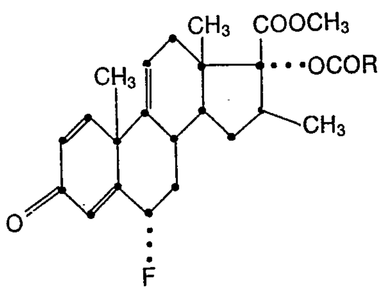

(III)

in which R has the meaning given above, or

c) a 1,2-saturated compound of the formula

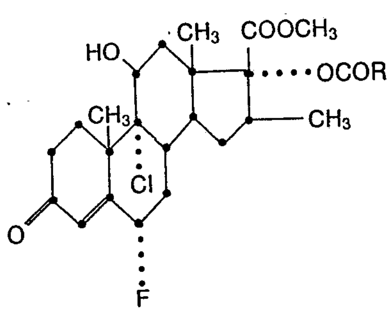

(IV)

in which R has the meaning given above is dehydrogenated in the 1,2-position, or

d) a carboxylic acid of the formula

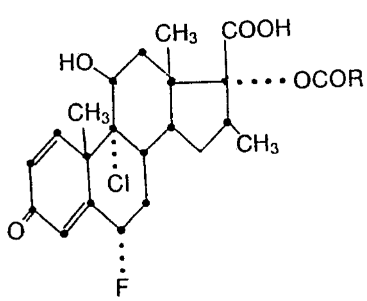

(V)

in which R has the meaning given above or a salt or a reactive functional derivative thereof is esterified using a methylating agent, or

e) 9α-chloro-6α-fluoro-11β,17α-dihydroxy-16β-methyl-3-oxo-androsta-1,4-diene-17β-carboxylic acid methyl ester of the formula

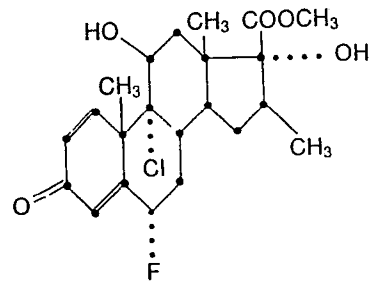

(VI)

is acylated with a carboxylic acid of the formula R–COOH (VII) in which R has the meaning given above, or with a reactive functional derivative thereof, optionally while temporarily protecting the 11β-hydroxy, or

f) in a compound of the formula

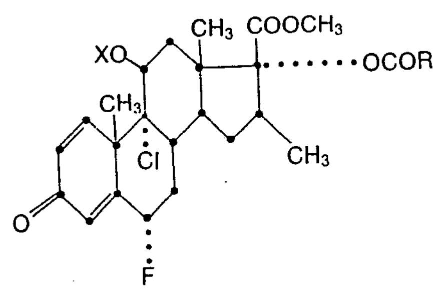

(VIII)

in which the 11β-hydroxy is protected and in which X represents a hydroxy-protecting group and R has the meaning given above, the protecting group X is removed.

9. A pharmaceutical composition containing at least one of the compounds mentioned in claims 1 to 7.

10. Use of a compound according to any one of claims 1 to 7 for the manufacture of an agent for the treatment of inflammations or allergies.

**Claims for the Contracting State: AT**

1. Process for the manufacture of a compound of the formula

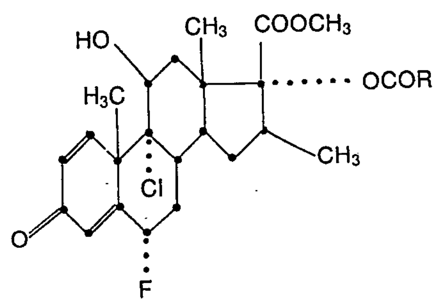

(A)

in which R represents alkyl having from 1 to 4 carbon atoms, characterised in that

a) a 9β,11β-oxido compound of the formula

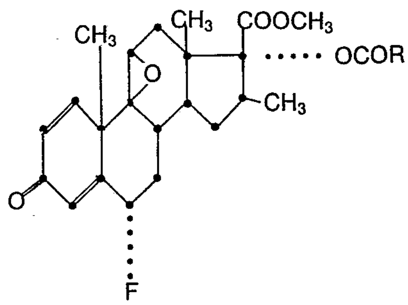

(II)

in which R has the meaning given above is treated with hydrogen chloride or with an agent yielding hydrogen chloride, or

b) elements of hypochlorous acid are added to a 9(11)-unsaturated compound of the formula

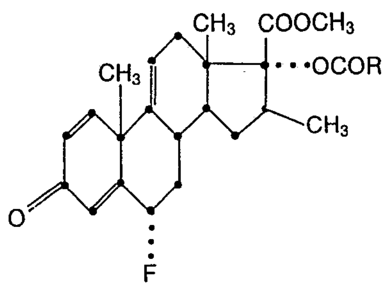

(III)

in which R has the meaning given above, or

c) a 1,2-saturated compound of the formula

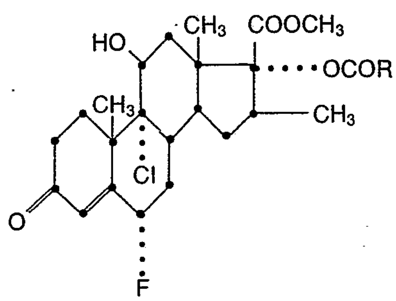

(IV)

in which R has the meaning given above is dehydrogenated in the 1,2-position, or

d) a carboxylic acid of the formula

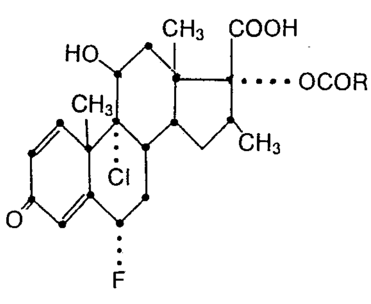

(V)

in which R has the meaning given above or a salt or a reactive functional derivative thereof is esterified using a methylating agent, or

e) 9α-chloro-6α-fluoro-11β,17α-dihydroxy-16β-methyl-3-oxo-androsta-1,4-diene-17β-carboxylic acid methyl ester of the formula

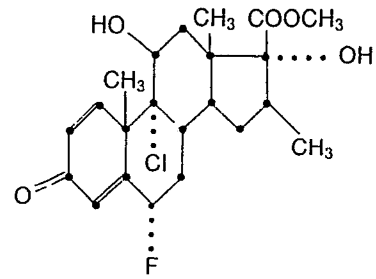

(VI)

is acylated with a carboxylic acid of the formula R–COOH (VII) in which R has the meaning given above, or with a reactive functional derivative thereof, optionally while temporarily protecting the 11β-hydroxy, or

f) in a compound of the formula

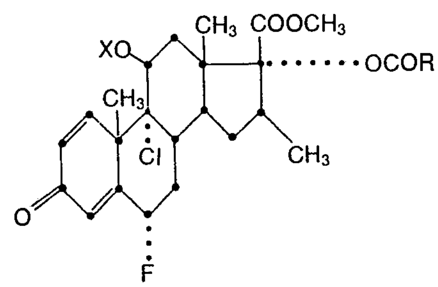

(VIII)

in which the 11β-hydroxy is protected and in which X represents a hydroxy-protecting group and R has the meaning given above, the protecting group X is removed.

2. Process according to claim 1, characterised in that there is manufactured a compound of the formula A in which R is a linear alkyl radical.

3. Process according to claim 1, characterised in that 9α-chloro-6α-fluoro-11β-hydroxy-16β-methyl-3-oxo-17α-propionyloxyandrosta-1,4-diene-17β-carboxylic acid methyl ester is manufactured.

4. Process according to claim 1, characterised in that 9α-chloro-6α-fluoro-11β-hydroxy-16β-methyl-3-oxo-17α-acetoxyandrosta-1,4-diene-17β-carboxylic acid methyl ester is manufactured.

5. Process according to claim 1, characterised in that 9α-chloro-6α-fluoro-11β-hydroxy-16β-methyl-3-oxo-17α-valeryloxyandrosta-1,4-diene-17β-carboxylic acid methyl ester is manufactured.

6. Process for the manufacture of pharmaceutical compositions containing at least one of the compounds mentioned in claims 1 to 5, characterised in that such a compound is mixed with at least one pharmaceutically acceptable adjunct.

**Revendications pour les états contractants: BE, CH, DE, FR, GB, IT, LI, LU, NL, SE**

1. Composés de formule

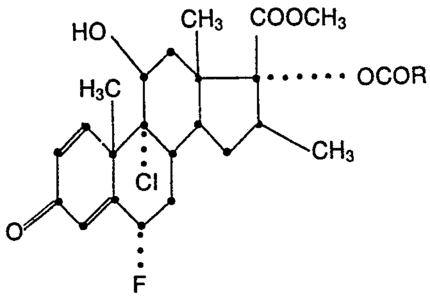

(A),

dans laquelle R représente un groupe alkyle en C 1–C 4.

2. Un composé de formule (A) selon la revendication 1, dans laquelle R représente un groupe alkyle linéaire.

3. Un composé selon la revendication 1, qui consiste en le 9 alpha-chloro-6 alpha-fluoro-11 bêta-hydroxy-16 bêta-méthyl-3-oxo-17 alpha-propionyloxy-androsta-1,4-diène-17 bêta-carboxylate de méthyle.

4. Un composé selon la revendication 1, qui consiste en le 9 alpha-chloro-6 alpha-fluoro-11 bêta-hydroxy-16 bêta-méthyl-3-oxo-17 alpha-acétoxy-androsta-1,4-diène-17 bêta-carboxylate de méthyle.

5. Un composé selon la revendication 1, qui consiste en le 9 alpha-chloro-6 alpha-fluoro-11 bêta-hydroxy-16 bêta-méthyl-3-oxo-17 alpha-valéryloxy-androsta-1,4-diène-17 bêta-carboxylate de méthyle.

6. Un composé selon l'une des revendications 1 à 5, pour l'utilisation thérapeutique.

7. Un composé selon l'une des revendications 1 à 5, pour le traitement des maladies de la peau et de l'asthme.

8. Procédé de préparation d'un composé de formule (A) selon l'une des revendications 1 à 5, caractérisé en ce que:

a) on traite un composé en 9 bêta, 11 bêta-oxydo, de formule

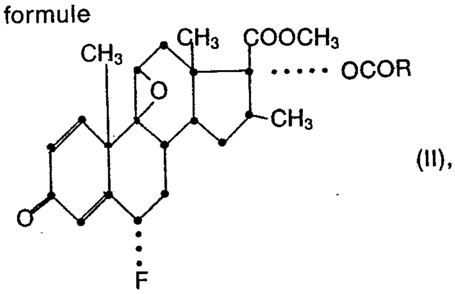

(II),

dans laquelle R a la signification indiquée dans l'une des revendications 1 à 5, par le chlorure d'hydrogène ou un agent libérant du chlorure d'hydrogène, ou bien

b) sur un composé à insaturation en 9(11), de formule

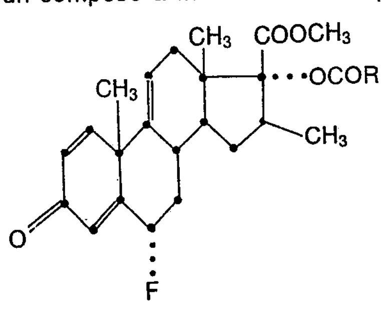

(III)

dans laquelle R a la signification indiquée ci-dessus, on fixe par addition les éléments de l'acide hypochloreux, ou bien

c) on soumet un composé saturé en 1,2, de formule

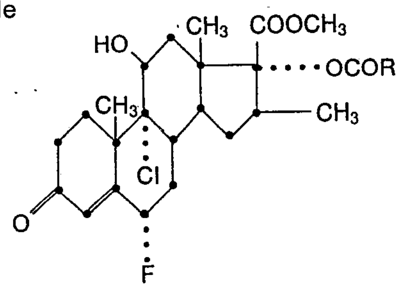

(IV)

dans laquelle R a la signification indiquée ci-dessus, à déshydrogénation en position 1,2, ou bien

d) on estérifie un acide carboxylique de formule

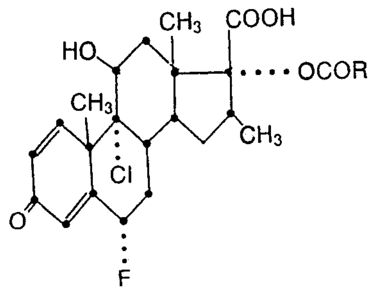

(V)

dans laquelle R a la signification indiquée ci-dessus, ou un sel ou un dérivé fonctionnel réactif d'un tel acide, à l'aide d'un agent méthylant, ou bien

e) on acyle le 9 alpha-chloro-6 alpha-fluoro-11 bêta, 17 alpha-dihydroxy-16 bêta-méthyl-3-oxo-androsta-1,4-diène-17 bêta-carboxylate de méthyle de formule

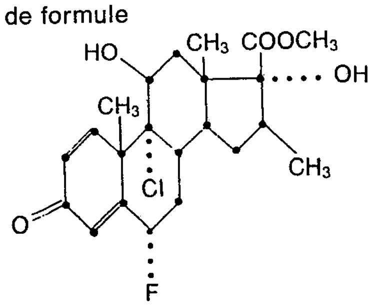

(VI)

par un acide carboxylique de formule R–COOH (VII) dans laquelle R a la signification indiquée ci-dessus, ou un dérivé réactif fonctionnel d'un tel acide, éventuellement en protégeant transitoirement le groupe 11 bêta-hydroxy, ou bien

f) dans un composé dans lequel le groupe 11 bêta-hydroxy est protégé, répondant à la formule

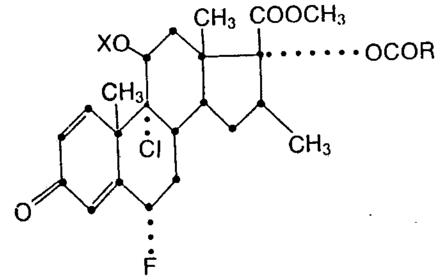

(VIII)

dans laquelle X représente un groupe protecteur du groupe hydroxy et R a la signification indiquée ci-dessus, on élimine le groupe protecteur X.

9. Une composition pharmaceutique contenant au moins un des composés mentionnés dans les revendications 1 à 7.

10. Utilisation d'un composé selon l'une des revendications 1 à 7 pour la préparation d'un produit pour le traitement des inflammations ou des allergies.

**Revendications pour l'état contractant: AT**

1. Procédé de préparation d'un composé de formule

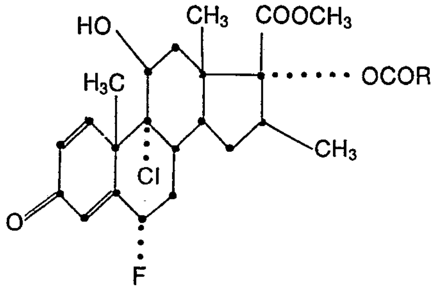

(A),

dans laquelle R représente un groupe alkyle en C 1–C 4, caractérisé en ce que:

a) on traite un composé en 9 bêta,11 bêta-oxydo de formule

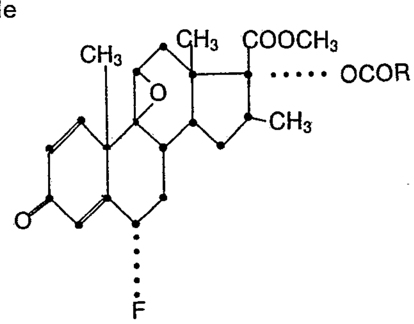

(II),

dans laquelle R a la signification indiquée ci-dessus, par le chlorure d'hydrogène ou un agent libérant du chlorure d'hydrogène, ou bien

b) sur un composé insaturé en 9(11) de formule

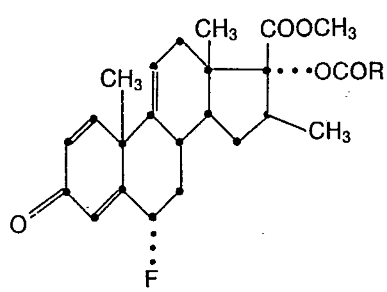

(III)

dans laquelle R a la signification indiquée ci-dessus, on fixe par addition les éléments de l'acide hypochloreux, ou bien

c) on soumet un composé saturé en 1,2, de formule

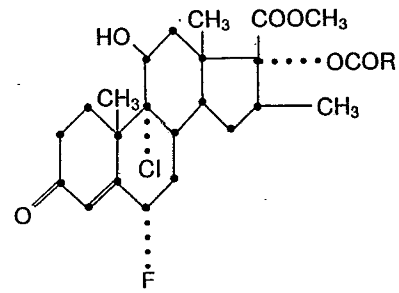

(IV)

dans laquelle R a la signification indiquée ci-dessus, à déshydrogénation en position 1,2, ou bien

d) on estérifie un acide carboxylique de formule

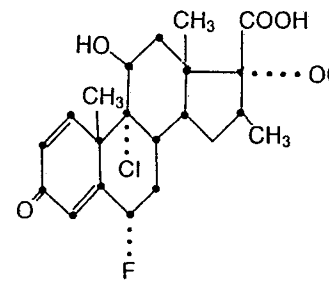

(V)

dans laquelle R a la signification indiquée ci-dessus, ou un sel ou un dérivé fonctionnel réactif d'un tel acide, à l'aide d'un agent méthylant, ou bien

e) on acyle le 9 alpha-chloro-6 alpha-fluoro-11 bêta, 17 alpha-dihydroxy-16 bêta-méthyl-3-oxo-androsta-1,4-diène-17 bêta-carboxylate de méthyle de formule

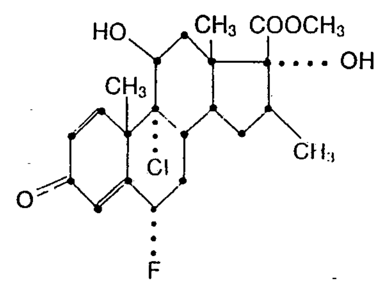

(VI)

à l'aide d'un acide carboxylique de formule R–COOH (VII) dans laquelle R a la signification indiquée ci-dessus, ou d'un dérivé fonctionnel réactif d'un tel acide, éventuellement en protégeant transitoirement le groupe 11 bêta-hydroxy, ou bien

f) dans un composé dans lequel le groupe 11 bêta-hydroxy est protégé, répondant à la formule

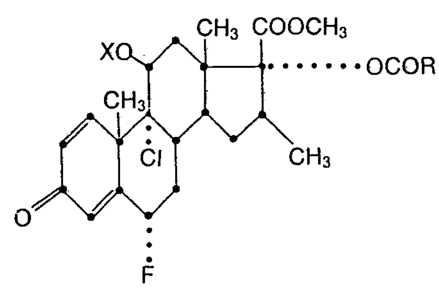

(VIII),

dans laquelle X représente un groupe protecteur

du groupe hydroxy et R a la signification indiquée ci-dessus, on élimine le groupe protecteur X.

2. Procédé selon la revendication 1, caractérisé en ce que l'on prépare un composé de formule (A) dans laquelle R représente un groupe alkyle linéaire.

3. Procédé selon la revendication 1, caractérisé en ce que l'on prépare le 9 alpha-chloro-6 alpha-fluoro-11 bêta-hydroxy-16 bêta-méthyl-3-oxo-17 alpha-propionyloxy-androsta-1,4-diène-17 bêta-carboxylate de méthyle.

4. Procédé selon la revendication 1, caractérisé en ce que l'on prépare le 9 alpha-chloro-6 alpha-fluoro-11 bêta-hydroxy-16 bêta-méthyl-3-oxo-17 alpha-acétoxy-androsta-1,4-diène-17 bêta-carboxylate de méthyle.

5. Procédé selon la revendication 1, caractérisé en ce que l'on prépare le 9 alpha-chloro-6 alpha-fluoro-11 bêta-hydroxy-16 bêta-méthyl-3-oxo-17 alpha-valéryloxy-androsta-1,4-diène-17 bêta-carboxylate de méthyle.

6. Procédé de préparation de compositions pharmaceutiques contenant au moins un des composés mentionnés dans les revendications 1 à 5, caractérisé en ce que l'on mélange un tel composé avec au moins un produit auxiliaire acceptable pour l'usage pharmaceutique.